# EUROPEAN PATENT APPLICATION

(11) **EP 3 862 443 A1**
(43) Date of publication of application: **11.08.2021**
(21) Application number: 19868650.3
(22) Date of filing: 27.09.2019
(51) Int. Cl.: C12Q 1/6888, C12Q 1/686, C12R 1/93

(54) **METHOD FOR DETECTING FISH PATHOGEN**

(30) Priority: 05.10.2018 US 201862741635 P
(71) Applicant: Schweitzer Biotech Company Ltd., Taipei, 11493 (TW)
(72) Inventor: KUO, Tsun-Yung, Zhuangwei Township I-Lan County, Taiwan 26341 (TW); HSIEH, Wang-Ju, Keelung City, Taiwan 20251 (TW); CHEN, Tai-An, Taipei City, Taiwan 10687 (TW); CHEN, Tz-Shiang, Taipei City, Taiwan 11493 (TW); HSIEH, Kai-Ru, Taipei City, Taiwan 11493 (TW); HSIAO, Chih-Chi, Taipei City, Taiwan 11493 (TW)
(74) Representative: Wittmann, Günther
(86) International application number: PCT/CN2019/108518
(87) International publication number: WO 2020/069656

(57) **Abstract**

A method for detecting a fish pathogen, and an oligonucleotide pair for detecting a fish pathogen.

## Description

### BACKGROUND OF THE INVENTION

### 1. FIELD OF THE INVENTION

The invention relates to a method for detecting pathogens in fish, more specifically to a method for detecting pathogens in fish using oligonucleotide pairs.

### 2. DESCRIPTION OF THE PRIOR ART

Aquatic animals are one of the important sources of human energy, protein, and essential nutrients. The concern about depletion of marine resources due to large-scale commercial fishing from wild fisheries makes the aquaculture industry more and more important. According to the statistics from the Food and Agriculture Organization of the United Nations, aquaculture has increasingly contributed to the total global production of fishing and aquaculture, from 25.7% in 2000 to 46.8% in 2016. However, intensive aquaculture and poor management may cause aquatic animals to be susceptible to diseases and death, and therefore, cause losses to fishermen. Most aquatic animal diseases are caused by bacteria and viruses. However, the treatments of bacterial diseases and viral diseases are very different, and therefore, misdiagnoses and improper treatments may cause huge losses in this industry. Thus, it is essential to intensely and accurately monitor and control diseases in fish and shrimp farms. The invention provides a fast and convenient method for detecting fish pathogens for industrial use.

### SUMMARY OF THE INVENTION

In one aspect, the invention relates to a method for detecting pathogens in fish, comprising providing a sample potentially containing one or more nucleotide sequences of a pathogen in fish, providing an oligonucleotide primer pair comprising a first primer and a second primer, and defining the 5' ends of two complementary strands of a double stranded target sequence on the one or more nucleotide sequences of the pathogen, providing a polymerase, blending the sample, the oligonucleotide primer pair, the polymerase, deoxyadenosine triphosphates (dATPs), deoxycytidine triphosphates (dCTPs), deoxyguanosine triphosphates (dGTPs), and deoxythymidine triphosphates (dTTPs) in a container to form a polymerase chain reaction (PCR) mixture, subjecting the PCR mixture to convective polymerase chain reaction (cPCR) by heating the bottom of the container at a fixed temperature to form a PCR product, and detecting the PCR product to identify the double stranded target sequence.

In some embodiments, the PCR mixture further comprises an oligonucleotide probe having a sequence complementary to a segment of the double stranded target sequence, a fluorescer molecule attached to a first location on the oligonucleotide probe, and a quencher molecule attached to a second location on the oligonucleotide probe such that the quencher molecule substantially quenches the fluorescer molecule whenever the oligonucleotide probe is not hybridized to the segment of the double stranded target sequence and such that the fluorescer molecule is substantially unquenched whenever the oligonucleotide probe is hybridized to the segment of the double stranded target sequence.

In another aspect, the invention relates to a pair of oligonucleotides for detecting pathogens in fish, comprising a first primer and a second primer.

In some embodiments, the pair of oligonucleotides further comprises an oligonucleotide probe having an oligonucleotide probe sequence, a fluorescer molecule attached to a first location on the oligonucleotide probe, and a quencher molecule attached to a second location on the oligonucleotide probe.

In some embodiments, the pathogen is piscine reovirus (PRV), and a sequence combination of the first primer and the second primer is selected from the group consisting of SEQ ID NO: 1 and SEQ ID NO: 2, SEQ ID NO: 4 and SEQ ID NO: 2, SEQ ID NO: 4 and SEQ ID NO: 5, SEQ ID NO: 6 and SEQ ID NO: 7, SEQ ID NO: 9 and SEQ ID NO: 10, and SEQ ID NO: 11 and SEQ ID NO: 12.

In some embodiments, the pathogen is PRV, and the sequence combination of the first primer and the second primer is SEQ ID NO: 1 and SEQ ID NO: 2, the oligonucleotide probe is a 13- to 25-bp oligonucleotide between the 71st to 95th nucleotides of segment L1 gene of PRV (GenBank accession No. KY429943). In some preferable embodiments, the oligonucleotide probe has a sequence of SEQ ID NO: 3.

In some embodiments, the pathogen is PRV, and the sequence combination of the first primer and the second primer is SEQ ID NO: 4 and SEQ ID NO: 2, the oligonucleotide probe is a 13- to 25-bp oligonucleotide between the 52nd to 95th nucleotides of segment L1 gene of PRV (GenBank accession No. KY429943). In some preferable embodiments, the oligonucleotide probe has a sequence of SEQ ID NO: 3.

In some embodiments, the pathogen is PRV, and the sequence combination of the first primer and the second primer is SEQ ID NO: 4 and SEQ ID NO: 5, the oligonucleotide probe is a 13- to 25-bp oligonucleotide between the 52nd to 100th nucleotides of segment L1 gene of PRV (GenBank accession No. KY429943). In some preferable embodiments, the oligonucleotide probe has a sequence of SEQ ID NO: 3.

In some embodiments, the pathogen is PRV, and the sequence combination of the first primer and the second primer is SEQ ID NO: 6 and SEQ ID NO: 7, the oligonucleotide probe is a 13- to 35-bp oligonucleotide between the 343rd to 385th nucleotides of the gene of Sigma 3 protein of PRV (GenBank accession No. KX844958). In some preferable embodiments, the oligonucleotide probe has a sequence of SEQ ID NO: 8.

In some embodiments, the pathogen is PRV, and the sequence combination of the first primer and the second primer is SEQ ID NO: 9 and SEQ ID NO: 10, the oligonucleotide probe is a 13- to 35-bp oligonucleotide between the 350th to 380th nucleotides of the gene of Sigma 3 protein of PRV (GenBank accession No. KX844958). In some preferable embodiments, the oligonucleotide probe has a sequence of SEQ ID NO: 8.

In some embodiments, the pathogen is PRV, and the sequence combination of the first primer and the second primer is SEQ ID NO: 11 and SEQ ID NO: 12, the oligonucleotide probe is a 13- to 35-bp oligonucleotide between the 280th to 315th nucleotides of segment L1 gene of PRV (GenBank accession No. KY429943). In some preferable embodiments, the oligonucleotide probe has a sequence of SEQ ID NO: 13.

In some embodiments, the pathogen is infectious pancreatic necrosis virus (IPNV) and a sequence combination of the first primer and the second primer is selected from the group consisting of SEQ ID NO: 14 and SEQ ID NO: 15, SEQ ID NO: 17 and SEQ ID NO: 18, SEQ ID NO: 20 and SEQ ID NO: 21, and SEQ ID NO: 23 and SEQ ID NO: 24.

In some embodiments, the pathogen is IPNV, and the sequence combination of the first primer and the second primer is SEQ ID NO: 14 and SEQ ID NO: 15, the oligonucleotide probe is a 13- to 35-bp oligonucleotide between the 2010th to 2057th nucleotides of VP5 and polyprotein genes of IPNV (GenBank accession No. KY548514). In some preferable embodiments, the oligonucleotide probe has a sequence of SEQ ID NO: 16.

In some embodiments, the pathogen is IPNV, and the sequence combination of the first primer and the second primer is SEQ ID NO: 17 and SEQ ID NO: 18, the oligonucleotide probe is a 13- to 35-bp oligonucleotide between the 2345th to 2408th nucleotides of VP5 and polyprotein genes of IPNV (GenBank accession No. KY548514). In some preferable embodiments, the oligonucleotide probe has a sequence of SEQ ID NO: 19.

In some embodiments, the pathogen is IPNV, and the sequence combination of the first primer and the second primer is SEQ ID NO: 20 and SEQ ID NO: 21, the oligonucleotide probe is a 13- to 35-bp oligonucleotide between the 2533rd to 2580th nucleotides of VP5 and polyprotein genes of IPNV (GenBank accession No. KY548514). In some preferable embodiments, the oligonucleotide probe has a sequence of SEQ ID NO: 22.

In some embodiments, the pathogen is IPNV, and the sequence combination of the first primer and the second primer is SEQ ID NO: 23 and SEQ ID NO: 24, the oligonucleotide probe is a 13- to 35-bp oligonucleotide between the 2775th to 2818th nucleotides of VP5 and polyprotein genes of IPNV (GenBank accession No. KY548514). In some preferable embodiments, the oligonucleotide probe has a sequence of SEQ ID NO: 25.

In some embodiments, the pathogen is infectious salmon anemia virus (ISAV) and a sequence combination of the first primer and the second primer is selected from the group consisting of SEQ ID NO: 26 and SEQ ID NO: 27, SEQ ID NO: 29 and SEQ ID NO: 30, SEQ ID NO: 32 and SEQ ID NO: 33, SEQ ID NO: 35 and SEQ ID NO: 36, and SEQ ID NO: 37 and SEQ ID NO: 38.

In some embodiments, the pathogen is ISAV, and the sequence combination of the first primer and the second primer is SEQ ID NO: 26 and SEQ ID NO: 27, the oligonucleotide probe is a 13- to 35-bp oligonucleotide between the 282nd to 328th nucleotides of open reading frame (ORF) 1 and ORF2 genes of ISAV (GenBank accession No. KX424589). In some preferable embodiments, the oligonucleotide probe has a sequence of SEQ ID NO: 28.

In some embodiments, the pathogen is ISAV, and the sequence combination of the first primer and the second primer is SEQ ID NO: 29 and SEQ ID NO: 30, the oligonucleotide probe is a 13- to 35-bp oligonucleotide between the 79th to 121st nucleotides of ORF1 and ORF2 genes of ISAV (GenBank accession No. KX424589). In some preferable embodiments, the oligonucleotide probe has a sequence of SEQ ID NO: 31.

In some embodiments, the pathogen is ISAV, and the sequence combination of the first primer and the second primer is SEQ ID NO: 32 and SEQ ID NO: 33, the oligonucleotide probe is a 13- to 30-bp oligonucleotide between the 244th to 273rd nucleotides of ORF1 and ORF2 genes of ISAV (GenBank accession No. KX424589). In some preferable embodiments, the oligonucleotide probe has a sequence of SEQ ID NO: 34.

In some embodiments, the pathogen is ISAV, and the sequence combination of the first primer and the second primer is SEQ ID NO: 35 and SEQ ID NO: 36, the oligonucleotide probe is a 13- to 35-bp oligonucleotide between the 237th to 272nd nucleotides of ORF1 and ORF2 genes of ISAV (GenBank accession No. KX424589). In some preferable embodiments, the oligonucleotide probe has a sequence of SEQ ID NO: 34.

In some embodiments, the pathogen is ISAV, and the sequence combination of the first primer and the second primer is SEQ ID NO: 37 and SEQ ID NO: 38, the oligonucleotide probe is a 13- to 35-bp oligonucleotide between the 235th to 286th nucleotides of ORF1 and ORF2 genes of ISAV (GenBank accession No. KX424589). In some preferable embodiments, the oligonucleotide probe has a sequence of SEQ ID NO: 34.

In some embodiments, the pathogen is piscine myocarditis virus (PMCV) and a sequence combination of the first primer and the second primer is selected from the group consisting of SEQ ID NO: 39 and SEQ ID NO: 40, SEQ ID NO: 42 and SEQ ID NO: 43, SEQ ID NO: 45 and SEQ ID NO: 46, SEQ ID NO: 48 and SEQ ID NO: 49, SEQ ID NO: 51 and SEQ ID NO: 49, and SEQ ID NO: 52 and SEQ ID NO: 49.

In some embodiments, the pathogen is PMCV, and the sequence combination of the first primer and the second primer is SEQ ID NO: 39 and SEQ ID NO: 40, the oligonucleotide probe is a 13- to 35-bp oligonucleotide between the 6266th to 6306th nucleotides of PMCV genome (GenBank accession No. HQ339954). In some preferable embodiments, the oligonucleotide probe has a sequence of SEQ ID NO: 41.

In some embodiments, the pathogen is PMCV, and the sequence combination of the first primer and the second primer is SEQ ID NO: 42 and SEQ ID NO: 43, the oligonucleotide probe is a 13- to 35-bp oligonucleotide between the 5632nd to 5672nd nucleotides of PMCV genome (GenBank accession No. HQ339954). In some preferable embodiments, the oligonucleotide probe has a sequence of SEQ ID NO: 44.

In some embodiments, the pathogen is PMCV, and the sequence combination of the first primer and the second primer is SEQ ID NO: 45 and SEQ ID NO: 46, the oligonucleotide probe is a 13- to 35-bp oligonucleotide between the 5693rd to 5747th nucleotides of PMCV genome (GenBank accession No. HQ339954). In some preferable embodiments, the oligonucleotide probe has a sequence of SEQ ID NO: 47.

In some embodiments, the pathogen is PMCV, and the sequence combination of the first primer and the second primer is SEQ ID NO: 48 and SEQ ID NO: 49, the oligonucleotide probe is a 13- to 35-bp oligonucleotide between the 6294th to 6331st nucleotides of PMCV genome (GenBank accession No. HQ339954). In some preferable embodiments, the oligonucleotide probe has a sequence of SEQ ID NO: 50.

In some embodiments, the pathogen is PMCV, and the sequence combination of the first primer and the second primer is SEQ ID NO: 51 and SEQ ID NO: 49, the oligonucleotide probe is a 13- to 35-bp oligonucleotide between the 6271st to 6331st nucleotides of PMCV genome (GenBank accession No. HQ339954). In some preferable embodiments, the oligonucleotide probe has a sequence of SEQ ID NO: 50.

In some embodiments, the pathogen is PMCV, and the sequence combination of the first primer and the second primer is SEQ ID NO: 52 and SEQ ID NO: 49, the oligonucleotide probe is a 13- to 35-bp oligonucleotide between the 6290th to 6331st nucleotides of PMCV genome (GenBank accession No. HQ339954). In some preferable embodiments, the oligonucleotide probe has a sequence of SEQ ID NO: 50.

In some embodiments, the pathogen is salmonid alphavirus (SAV) and a sequence combination of the first primer and the second primer is selected from the group consisting of SEQ ID NO: 53 and SEQ ID NO: 54, SEQ ID NO: 56 and SEQ ID NO: 57, SEQ ID NO: 59 and SEQ ID NO: 60, SEQ ID NO: 62 and SEQ ID NO: 63, and SEQ ID NO: 65 and SEQ ID NO: 66.

In some embodiments, the pathogen is SAV, and the sequence combination of the first primer and the second primer is SEQ ID NO: 53 and SEQ ID NO: 54, the oligonucleotide probe is a 13- to 35-bp oligonucleotide between the 611st to 653rd nucleotides of SAV genome (GenBank accession No. KC122926). In some preferable embodiments, the oligonucleotide probe has a sequence of SEQ ID NO: 55.

In some embodiments, the pathogen is SAV, and the sequence combination of the first primer and the second primer is SEQ ID NO: 56 and SEQ ID NO: 57, the oligonucleotide probe is a 13- to 35-bp oligonucleotide between the 145th to 181st nucleotides of SAV genome (GenBank accession No. KC122926). In some preferable embodiments, the oligonucleotide probe has a sequence of SEQ ID NO: 58.

In some embodiments, the pathogen is SAV, and the sequence combination of the first primer and the second primer is SEQ ID NO: 59 and SEQ ID NO: 60, the oligonucleotide probe is a 13- to 35-bp oligonucleotide between the 2691st to 2761st nucleotides of SAV genome (GenBank accession No. KC122926). In some preferable embodiments, the oligonucleotide probe has a sequence of SEQ ID NO: 61.

In some embodiments, the pathogen is SAV, and the sequence combination of the first primer and the second primer is SEQ ID NO: 62 and SEQ ID NO: 63, the oligonucleotide probe is a 13- to 35-bp oligonucleotide between the 1032nd to 1075th nucleotides of SAV genome (GenBank accession No. KC122926). In some preferable embodiments, the oligonucleotide probe has a sequence of SEQ ID NO: 64.

In some embodiments, the pathogen is SAV, and the sequence combination of the first primer and the second primer is SEQ ID NO: 65 and SEQ ID NO: 66, the oligonucleotide probe is a 13- to 35-bp oligonucleotide between the 969th to 1011st nucleotides of SAV genome (GenBank accession No. KC122926). In some preferable embodiments, the oligonucleotide probe has a sequence of SEQ ID NO: 67.

In some embodiments, the pathogen is *Neoparamoebaperurans* and a sequence combination of the first primer and the second primer is selected from the group consisting of SEQ ID NO: 68 and SEQ ID NO: 69, SEQ ID NO: 71 and SEQ ID NO: 72, SEQ ID NO: 74 and SEQ ID NO: 75, and SEQ ID NO: 77 and SEQ ID NO: 78.

In some embodiments, the pathogen is *N. perurans,* and the sequence combination of the first primer and the second primer is SEQ ID NO: 68 and SEQ ID NO: 69, the oligonucleotide probe is a 13- to 35-bp oligonucleotide between the 462nd to 520th nucleotides of 18S ribosomal RNA (rRNA) of *N. perurans* (GenBank accession No. KU985058). In some preferable embodiments, the oligonucleotide probe has a sequence of SEQ ID NO: 70.

In some embodiments, the pathogen is *N. perurans,* and the sequence combination of the first primer and the second primer is SEQ ID NO: 71 and SEQ ID NO: 72, the oligonucleotide probe is a 13- to 35-bp oligonucleotide between the 355th to 405th nucleotides of 18S rRNA of *N. perurans* (GenBank accession No. KU985058). In some preferable embodiments, the oligonucleotide probe has a sequence of SEQ ID NO: 73.

In some embodiments, the pathogen is *N. perurans,* and the sequence combination of the first primer and the second primer is SEQ ID NO: 74 and SEQ ID NO: 75, the oligonucleotide probe is a 13- to 35-bp oligonucleotide between the 234th to 281st nucleotides of 18S rRNA of *N. perurans* (GenBank accession No. KU985058). In some preferable embodiments, the oligonucleotide probe has a sequence of SEQ ID NO: 76.

In some embodiments, the pathogen is *N. perurans,* and the sequence combination of the first primer and the second primer is SEQ ID NO: 77 and SEQ ID NO: 78, the oligonucleotide probe is a 13- to 24-bp oligonucleotide between the 357th to 380th nucleotides of 18S rRNA of *N. perurans* (GenBank accession No. KU985058). In some preferable embodiments, the oligonucleotide probe has a sequence of SEQ ID NO: 79.

In some embodiments, the pathogen is *Candidatus Branchiomonas cysticola* (CBc) and a sequence combination of the first primer and the second primer is selected from the group consisting of SEQ ID NO: 80 and SEQ ID NO: 81, and SEQ ID NO: 83 and SEQ ID NO: 84.

In some embodiments, the pathogen is *Ca. B. cysticola* (CBc), and the sequence combination of the first primer and the second primer is SEQ ID NO: 80 and SEQ ID NO: 81, the oligonucleotide probe is a 13- to 35-bp oligonucleotide between the 1068th to 1125th nucleotides of 16S ribosomal RNA (rRNA) of CBc (GenBank accession No. JQ723599). In some preferable embodiments, the oligonucleotide probe has a sequence of SEQ ID NO: 82.

In some embodiments, the pathogen is *Ca. B. cysticola* (CBc), and the sequence combination of the first primer and the second primer is SEQ ID NO: 83 and SEQ ID NO: 84, the oligonucleotide probe is a 13- to 30-bp oligonucleotide between the 1212nd to 1241th nucleotides of 16S rRNA of CBc (GenBank accession No. JQ723599). In some preferable embodiments, the oligonucleotide probe has a sequence of SEQ ID NO: 85.

In some embodiments, the pathogen is *Desmozoon lepeophtherii* (Des) and a sequence combination of the first primer and the second primer is selected from the group consisting of SEQ ID NO: 86 and SEQ ID NO: 87, SEQ ID NO: 89 and SEQ ID NO: 90, SEQ ID NO: 92 and SEQ ID NO: 93, SEQ ID NO: 95 and SEQ ID NO: 97, and SEQ ID NO: 96 and SEQ ID NO: 97.

In some embodiments, the pathogen is *D. lepeophtherii* (Des), and the sequence combination of the first primer and the second primer is SEQ ID NO: 86 and SEQ ID NO: 87, the oligonucleotide probe is a 13- to 35-bp oligonucleotide between the 967th to 1010th nucleotides of 16S ribosomal RNA (rRNA) of Des (GenBank accession No. AJ431366). In some preferable embodiments, the oligonucleotide probe has a sequence of SEQ ID NO: 88.

In some embodiments, the pathogen is *D. lepeophtherii* (Des), and the sequence combination of the first primer and the second primer is SEQ ID NO: 89 and SEQ ID NO: 90, the oligonucleotide probe is a 13- to 35-bp oligonucleotide between the 1246th to 1285th nucleotides of 16S rRNA of Des (GenBank accession No. AJ431366). In some preferable embodiments, the oligonucleotide probe has a sequence of SEQ ID NO: 91.

In some embodiments, the pathogen is *D. lepeophtherii* (Des), and the sequence combination of the first primer and the second primer is SEQ ID NO: 92 and SEQ ID NO: 93, the oligonucleotide probe is a 13- to 35-bp oligonucleotide between the 1366th to 1414th nucleotides of 16S rRNA of Des (GenBank accession No. AJ431366). In some preferable embodiments, the oligonucleotide probe has a sequence of SEQ ID NO: 94.

In some embodiments, the pathogen is *D. lepeophtherii* (Des), and the sequence combination of the first primer and the second primer is SEQ ID NO: 95 and SEQ ID NO: 97, the oligonucleotide probe is a 13- to 35-bp oligonucleotide between the 815th to 859th nucleotides of 16S rRNA of Des (GenBank accession No. AJ431366). In some preferable embodiments, the oligonucleotide probe has a sequence of SEQ ID NO: 98.

In some embodiments, the pathogen is *D. lepeophtherii* (Des), and the sequence combination of the first primer and the second primer is SEQ ID NO: 96 and SEQ ID NO: 97, the oligonucleotide probe is a 13- to 35-bp oligonucleotide between the 817th to 859th nucleotides of 16S rRNA of Des (GenBank accession No. AJ431366). In some preferable embodiments, the oligonucleotide probe has a sequence of SEQ ID NO: 98.

In some embodiments, the pathogen is salmon gill poxvirus (SGPV) and a sequence combination of the first primer and the second primer is selected from the group consisting of SEQ ID NO: 99 and SEQ ID NO: 100, and SEQ ID NO: 102 and SEQ ID NO: 103, and SEQ ID NO: 105 and SEQ ID NO: 106, and SEQ ID NO: 108 and SEQ ID NO: 109.

In some embodiments, the pathogen is SGPV, and the sequence combination of the first primer and the second primer is SEQ ID NO: 99 and SEQ ID NO: 100, the oligonucleotide probe is a 13- to 35-bp oligonucleotide between the 99272nd to 99318th nucleotides of SGPV genome (GenBank accession No. KT159937). In some preferable embodiments, the oligonucleotide probe has a sequence of SEQ ID NO: 101.

In some embodiments, the pathogen is SGPV, and the sequence combination of the first primer and the second primer is SEQ ID NO: 102 and SEQ ID NO: 103, the oligonucleotide probe is a 13- to 27-bp oligonucleotide between the 123213rd to 123239th nucleotides of SGPV genome (GenBank accession No. KT159937). In some preferable embodiments, the oligonucleotide probe has a sequence of SEQ ID NO: 104.

In some embodiments, the pathogen is SGPV, and the sequence combination of the first primer and the second primer is SEQ ID NO: 105 and SEQ ID NO: 106, the oligonucleotide probe is a 13- to 30-bp oligonucleotide between the 123202nd to 123232nd nucleotides of SGPV genome (GenBank accession No. KT159937). In some preferable embodiments, the oligonucleotide probe has a sequence of SEQ ID NO: 107.

In some embodiments, the pathogen is SGPV, and the sequence combination of the first primer and the second primer is SEQ ID NO: 108 and SEQ ID NO: 109, the oligonucleotide probe is a 13- to 35-bp oligonucleotide between the 123556th to 123610th nucleotides of SGPV genome (GenBank accession No. KT159937). In some preferable embodiments, the oligonucleotide probe has a sequence of SEQ ID NO: 110.

In some embodiments, the pathogen is *Piscirickettsia salmonis* and a sequence combination of the first primer and the second primer is selected from the group consisting of SEQ ID NO: 111 and SEQ ID NO: 113, and SEQ ID NO: 114 and SEQ ID NO: 115, and SEQ ID NO: 116 and SEQ ID NO: 117, and SEQ ID NO: 119 and SEQ ID NO: 120, and SEQ ID NO: 122 and SEQ ID NO: 123.

In some embodiments, the pathogen is *P. salmonis,* and the sequence combination of the first primer and the second primer is SEQ ID NO: 111 and SEQ ID NO: 112, the oligonucleotide probe is a 13- to 35-bp oligonucleotide between the 952nd to 1065th nucleotides of 16S ribosomal RNA (rRNA) of *P. salmonis* (GenBank accession No. AY498634). In some preferable embodiments, the oligonucleotide probe has a sequence of SEQ ID NO: 113.

In some embodiments, the pathogen is *P. salmonis,* and the sequence combination of the first primer and the second primer is SEQ ID NO: 114 and SEQ ID NO: 115, the oligonucleotide probe is a 13- to 35-bp oligonucleotide between the 1014th to 1050th nucleotides of 16S rRNA of *P. salmonis* (GenBank accession No. AY498634). In some preferable embodiments, the oligonucleotide probe has a sequence of SEQ ID NO: 113.

In some embodiments, the pathogen is *P. salmonis,* and the sequence combination of the first primer and the second primer is SEQ ID NO: 116 and SEQ ID NO: 117, the oligonucleotide probe is a 13- to 17-bp oligonucleotide between the 315th to 331st nucleotides of 16S rRNA of *P. salmonis* (GenBank accession No. AY498634). In some preferable embodiments, the oligonucleotide probe has a sequence of SEQ ID NO: 118.

In some embodiments, the pathogen is *P. salmonis,* and the sequence combination of the first primer and the second primer is SEQ ID NO: 119 and SEQ ID NO: 120, the oligonucleotide probe is a 13- to 17-bp oligonucleotide between the 529th to 545th nucleotides of 16S rRNA of *P. salmonis* (GenBank accession No. AY498634). In some preferable embodiments, the oligonucleotide probe has a sequence of SEQ ID NO: 121.

In some embodiments, the pathogen is *P. salmonis,* and the sequence combination of the first primer and the second primer is SEQ ID NO: 122 and SEQ ID NO: 123, the oligonucleotide probe is a 13- to 20-bp oligonucleotide between the 1181st to 1200th nucleotides of 16S rRNA of *P. salmonis* (GenBank accession No. AY498634). In some preferable embodiments, the oligonucleotide probe has a sequence of SEQ ID NO: 124.

In some embodiments, the pathogen is *Renibacterium salmoninarum* and a sequence combination of the first primer and the second primer is selected from the group consisting of SEQ ID NO: 126 and SEQ ID NO: 128, and SEQ ID NO: 127 and SEQ ID NO: 128, and SEQ ID NO: 130 and SEQ ID NO: 131, and SEQ ID NO: 133 and SEQ ID NO: 134, and SEQ ID NO: 136 and SEQ ID NO: 137.

In some embodiments, the pathogen is *R. salmoninarum,* and the sequence combination of the first primer and the second primer is SEQ ID NO: 125 and SEQ ID NO: 128, the oligonucleotide probe is a 13- to 31-bp oligonucleotide between the 1035th to 1065th nucleotides of 57KD extracellular protein precursor (ECP) of *R. salmoninarum* (GenBank accession No. Z12174). In some preferable embodiments, the oligonucleotide probe has a sequence of SEQ ID NO: 129.

In some embodiments, the pathogen is *R. salmoninarum,* and the sequence combination of the first primer and the second primer is SEQ ID NO: 126 and SEQ ID NO: 128, the oligonucleotide probe is a 13- to 35-bp oligonucleotide between the 1024th to 1065th nucleotides of 57KD ECP of *R. salmoninarum* (GenBank accession No. Z12174). In some preferable embodiments, the oligonucleotide probe has a sequence of SEQ ID NO: 129.

In some embodiments, the pathogen is *R. salmoninarum,* and the sequence combination of the first primer and the second primer is SEQ ID NO: 127 and SEQ ID NO: 128, the oligonucleotide probe is a 13- to 29-bp oligonucleotide between the 1037th to 1065th nucleotides of 57KD ECP of *R. salmoninarum* (GenBank accession No. Z12174). In some preferable embodiments, the oligonucleotide probe has a sequence of SEQ ID NO: 129.

In some embodiments, the pathogen is *R. salmoninarum,* and the sequence combination of the first primer and the second primer is SEQ ID NO: 130 and SEQ ID NO: 131, the oligonucleotide probe is a 13- to 25-bp oligonucleotide between the 782nd to 806th nucleotides of 57KD ECP of *R. salmoninarum* (GenBank accession No. Z12174). In some preferable embodiments, the oligonucleotide probe has a sequence of SEQ ID NO: 132.

In some embodiments, the pathogen is *R. salmoninarum,* and the sequence combination of the first primer and the second primer is SEQ ID NO: 133 and SEQ ID NO: 134, the oligonucleotide probe is a 13- to 35-bp oligonucleotide between the 880th to 964th nucleotides of 57KD ECP of *R. salmoninarum* (GenBank accession No. Z12174). In some preferable embodiments, the oligonucleotide probe has a sequence of SEQ ID NO: 135.

In some embodiments, the pathogen is *R. salmoninarum,* and the sequence combination of the first primer and the second primer is SEQ ID NO: 136 and SEQ ID NO: 137, the oligonucleotide probe is a 13- to 35-bp oligonucleotide between the 646th to 738th nucleotides of 57KD ECP of *R. salmoninarum* (GenBank accession No. Z12174). In some preferable embodiments, the oligonucleotide probe has a sequence of SEQ ID NO: 138.

Unless defined otherwise, all the technical and scientific terms used herein have the same meanings as are commonly understood by one of skill in the art to which this invention belongs.

The present invention is described in more detail in the following illustrative examples. Although the examples may represent only selected embodiments of the invention, it should be understood that the following examples are illustrative and not limiting.

### DETAILED DESCRIPTION OF THE PREFERRED EMBODIMENT

In one aspect, the invention relates to a method for detecting a pathogen in fish. In some embodiments, the method is polymerase chain reaction (PCR). In some embodiments, the method is reverse-transcription polymerase chain reaction (RT-PCR). In some embodiments, the method is convective polymerase chain reaction, (cPCR). In some embodiments, the method is real-time quantitative polymerase chain reaction (real-time PCR).

In one aspect, the invention relates to a method for detecting a pathogen in fish, comprising:
providing a sample potentially containing one or more nucleotide sequences of a pathogen in fish;
providing an oligonucleotide primer pair comprising a first primer and a second primer, and defining the 5' ends of two complementary strands of a double stranded target sequence on the one or more nucleotide sequences of the pathogen;
providing a polymerase;
blending the sample, the oligonucleotide primer pair, the polymerase, deoxyadenosine triphosphates (dATPs), deoxycytidine triphosphates (dCTPs), deoxyguanosine triphosphates (dGTPs), and deoxythymidine triphosphates (dTTPs) in a container to form a polymerase chain reaction (PCR) mixture;
subjecting the PCR mixture to convective polymerase chain reaction (cPCR) by heating the bottom of the container at a fixed temperature to form a PCR product; and
detecting the PCR product to identify the double stranded target sequence.

In another aspect, the invention relates to a method for detecting a pathogen in fish, comprising:
providing a sample potentially containing one or more nucleotide sequences of a pathogen in fish;
providing an oligonucleotide primer pair comprising a first primer and a second primer, and defining the 5' ends of two complementary strands of a double stranded target sequence on the one or more nucleotide sequences of the pathogen;
providing an oligonucleotide probe having a sequence complementary to a segment of the double stranded target sequence, a fluorescer molecule attached to a first location on the oligonucleotide probe, and a quencher molecule attached to a second location on the oligonucleotide probe such that the quencher molecule substantially quenches the fluorescer molecule whenever the oligonucleotide probe is not hybridized to the segment of the double stranded target sequence and such that the fluorescer molecule is substantially unquenched whenever the oligonucleotide probe is hybridized to the segment of the double stranded target sequence;
providing a polymerase;
blending the sample, the oligonucleotide primer pair, the oligonucleotide probe, the polymerase, deoxyadenosine triphosphates (dATPs), deoxycytidine triphosphates (dCTPs), deoxyguanosine triphosphates (dGTPs), and deoxythymidine triphosphates (dTTPs) in a container to form a polymerase chain reaction (PCR) mixture;
subjecting the PCR mixture to convective polymerase chain reaction (cPCR) by heating the bottom of the container at a fixed temperature to form a PCR product; and
detecting the PCR product to identify the double stranded target sequence.

In a further aspect, the invention relates to a pair of oligonucleotides for detecting pathogens in fish.

In yet another aspect, the invention relates to a pair of oligonucleotides and an oligonucleotide probe for detecting pathogens in fish.

It should further be appreciated that in some embodiments, a pair of oligonucleotides and/or an oligonucleotide probe disclosed herein may be used in variations on the basic PCR technique, such as but not limited to, reverse-transcription PCR (RT-PCR), convective polymerase chain reaction (cPCR), real-time quantitative PCR (real-time PCR), nested PCR, and thermal asymmetric interlaced PCR (TAIL-PCR).

As used herein, the term "a pathogen in fish" refers to viral, bacterial and parasitic pathogen found in fish. Examples of pathogens in fish are such as but not limited to piscine reovirus (PRV), infectious pancreatic necrosis virus (IPNV), infectious salmon anemia virus (ISAV), piscine myocarditis virus (PMCV), salmonid alphavirus (SAV), *Neoparamoeba perurans, Candidatus Branchiomonas cysticola* (CBc), *Desmozoon lepeophtherii* (Des), salmon gill poxvirus (SGPV), *Piscirickettsia salmonis,* and *Renibacterium salmoninarum.*

As used herein, the term "convective polymerase chain reaction" (cPCR) refers to a polymerase chain reaction in which the bottom of a tubular container containing a PCR sample is embedded in a stable heat source, and the PCR parameters, including the total volume, viscosity, surface temperature of the PCR sample, and the inner diameter of the tubular container, are controlled so that the temperature gradient from the bottom to the top of the PCR sample decreases, which induces thermal convection and causes denaturation, annealing, and extension of the PCR sample to occur sequentially and repeatedly in different sections of the tubular container. For a detailed description of the convective PCR, see e.g., U.S. Patent Nos. 8,187,813, which is incorporated herein by reference in their entireties.

As used herein, the term "fluorescer molecule" refers to a substance or a portion thereof which is capable of exhibiting fluorescence in the detectable range. As used herein, the term "quencher molecule" refers to a substance or a portion thereof which is capable of quenching the fluorescence emitted by the fluorescer molecule when excited by a light source. In some embodiments, the terms "fluorescer molecule" and "quencher molecule" are the fluorescer molecule and the quencher molecule of the TaqMan™ assay (Applied Biosystems Inc., CA, US). For a detailed description of the TaqMan™ assay, reagents, and conditions for use therein, see, e.g., Holland et al., Proc. Natl. Acad. Sci, U.S.A. (1991) 88:7276- 7280; U.S. Patent Nos. 5,538,848, 5,723,591, 5,876,930, and 7,413,708 all incorporated herein by reference in their entireties.

Example of the fluorescer molecule includes, but not limited to, 3-(ε-carboxypentyl)-3'-ethyl-5,5'-dimethyloxa-carbocyanine (CYA); 6-carboxyfluorescein (FAM); 5,6-carboxyrhodamine-11O (R110); 6- carboxyrhodamine-6G (R6G); *N',N',N',N'-*tetramethyl-6-carboxyrhodamine (TAMRA); 6-carboxy-X-rhodamine (ROX); 2',4',5',7'-tetrachloro-4-7-dichlorofluorescein (TET); 2',7- dimethoxy -4',5'-6carboxyrhodamine (JOE); 6- carboxy-2',4,4',5',7,7'-hexachlorofluorescein (HEX); ALEXA; Cy3 and Cy5. Example of the quencher molecule includes, but not limited to, 4-(4'-dimethylamino-phenylazo)-benzoic acid (Dabcyl), Black Hole Quencher 1 (BHQ1), Black Hole Quencher 2 (BHQ2), Black Hole Quencher 3 (BHQ3), tetramethylrhodamine (TAMRA). In some embodiments, the fluorescer molecule is 6-carboxyfluorescein (6-FAM), and the quencher molecule is dihydrocyclopyrroloindole tripeptide minor groove binder (MGB).

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well-known in the art. Generally, nomenclatures used in connection with, and techniques of biochemistry, enzymology, molecular and cellular biology, microbiology, genetics and protein and nucleic acid chemistry and hybridization described herein are those well-known and commonly used in the art. The methods and techniques of the present disclosure are generally performed according to conventional methods well known in the art and as described in various general and more specific references that are cited and discussed throughout the present specification unless otherwise indicated.

The present invention is further illustrated by the following Examples, which in no way should be construed as further limiting. The entire contents of all of the references (including literature references, issued patents, published patent applications, and co-pending patent applications) cited throughout this application are hereby expressly incorporated by reference.

### EXAMPLES

### Example 1 Detection of Piscine Reovirus (PRV)

The segment L1 gene of PRV (GenBank accession No. KY429943) and the gene of Sigma 3 protein of PRV (GenBank accession No. KX844958) were inserted in a cloning vector to obtain pPRV-L1 and pPRV-S3 plasmids, respectively. The cloning vector can be, but is not limited to, pUC57 and pGEM-T.

### 1. Conventional PCR

The 50 µl PCR mixture for conventional PCR contains 10⁶ copies of pPRV-L1 or pPRV-S3 plasmids, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer (40 mM Tris, 20 mM acetic acid, 1 mM EDTA) and visualized by ethidium bromide staining.

The results of the conventional PCR are shown in Table 1, indicating that each primer pair of the invention has correctly amplified the target sequence, whereas no target sequence has been amplified in the negative control (data not shown). The results demonstrate that the primer pairs of the invention can be used in conventional PCR amplification to detect the existence of PRV.

**Table 1 The results of the conventional PCR**

| Forward Primer | Reverse Primer | Size of Target Sequence (bp) |
|---|---|---|
| PRV-F1 (SEQ ID NO: 1) | PRV-R1 (SEQ ID NO: 2) | 70 |
| PRV-F2 (SEQ ID NO: 4) | PRV-R1 (SEQ ID NO: 2) | 92 |
| PRV-F2 (SEQ ID NO: 4) | PRV-R2 (SEQ ID NO: 5) | 98 |
| PRV-F3 (SEQ ID NO: 6) | PRV-R3 (SEQ ID NO: 7) | 83 |
| PRV-F4 (SEQ ID NO: 9) | PRV-R4 (SEQ ID NO: 10) | 77 |
| PRV-F5 (SEQ ID NO: 11) | PRV-R5 (SEQ ID NO: 12) | 76 |

### 2. Convective PCR (cPCR)

The 50 µl PCR mixture, containing the mRNA (10², 10³, 10⁴, 10⁵, 10⁶ copies/µl respectively) of the segment L1 gene of PRV or the mRNA (10², 10³, 10⁴, 10⁵, 10⁶ copies/µl respectively) of the gene of Sigma 3 protein of PRV, or pPRV-L1 or pPRV-S3 plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 2, indicating that each combination of the primer pair and the probe of the invention has 100% correctly detected samples that contain 10² copies/µl of the mRNA of the segment L1 gene of PRV or the gene of Sigma 3 protein of PRV, or 10² copies of pPRV-L1 or pPRV-S3 plasmid. The sensitivity of each primer pair and probe is 10² copies/µl and 10² copies.

**Table 2 The cPCR sensitivity of each combination of primer pair and probe (n=8)**

| Combination of Primer Pair and Probe Positive Rate (%) | NC* | mRNA content of the segment L1 gene or the gene of Sigma 3 protein of PRV (copies/µl) | | | | | pPRV-L1 or pPRV-S3 plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| PRV-F1 (SEQ ID NO: 1) | 0 | 100 | 100 | 100 | 100 | 100 | 100 | 100 | 100 | 100 | 100 |
| PRV-R1 (SEQ ID NO: 2) | | | | | | | | | | | |
| PRV-P1 (SEQ ID NO: 3) | | | | | | | | | | | |
| PRV-F2 (SEQ ID NO: 4) | 0 | 100 | 100 | 100 | 100 | 100 | 100 | 100 | 100 | 100 | 100 |
| PRV-R1 (SEQ ID NO: 2) | | | | | | | | | | | |
| PRV-P1 (SEQ ID NO: 3) | | | | | | | | | | | |
| PRV-F2 (SEQ ID NO: 4) | 0 | 100 | 100 | 100 | 100 | 100 | 100 | 100 | 100 | 100 | 100 |
| PRV-R2 (SEQ ID NO: 5) | | | | | | | | | | | |
| PRV-P1 (SEQ ID NO: 3) | | | | | | | | | | | |
| PRV-F3 (SEQ ID NO: 6) | 0 | 100 | 100 | 100 | 100 | 100 | 100 | 100 | 100 | 100 | 100 |
| PRV-R3 (SEQ ID NO: 7) | | | | | | | | | | | |
| PRV-P3 (SEQ ID NO: 8) | | | | | | | | | | | |
| PRV-F4 (SEQ ID NO: 9) | 0 | 100 | 100 | 100 | 100 | 100 | 100 | 100 | 100 | 100 | 100 |
| PRV-R4 (SEQ ID NO: 10) | | | | | | | | | | | |
| PRV-P3 (SEQ ID NO: 8) | | | | | | | | | | | |
| PRV-F5 (SEQ ID NO: 11) | 0 | 100 | 100 | 100 | 100 | 100 | 100 | 100 | 100 | 100 | 100 |
| PRV-R5 (SEQ ID NO: 12) | | | | | | | | | | | |
| PRV-P5 (SEQ ID NO: 13) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 3, indicating that each combination of primer pair and probe of the invention has correctly detected samples that contain PRV but not samples that contain IPNV, ISAV, PMCV, SAV, *N. perurans* (indicated with AGD in Table 3), *C. Branchiomonas cysticola* (CBc), *D. lepeophtherii* (Des), SGPV, *P. salmonis* (indicated with SRS in Table 3), and *R. salmoninarum* (indicated with BKD in Table 3). The results demonstrate that all the combinations of the primer pairs and the probes of the invention is specific to PRV.

**Table 3 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| PRV-F1 (SEQ ID NO: 1) | + | - | - | - | - | - | - | - | - | - | - |
| PRV-R1 (SEQ ID NO: 2) | | | | | | | | | | | |
| PRV-P1 (SEQ ID NO: 3) | | | | | | | | | | | |
| PRV-F2 (SEQ ID NO: 4) | + | - | - | - | - | - | - | - | - | - | - |
| PRV-R1 (SEQ ID NO: 2) | | | | | | | | | | | |
| PRV-P1 (SEQ ID NO: 3) | | | | | | | | | | | |
| PRV-F2 (SEQ ID NO: 4) | | - | - | - | - | - | - | - | - | - | - |
| PRV-R2 (SEQ ID NO: 5) | + | | | | | | | | | | |
| PRV-P1 (SEQ ID NO: 3) | | | | | | | | | | | |
| PRV-F3 (SEQ ID NO: 6) | + | - | - | - | - | - | - | - | - | - | - |
| PRV-R3 (SEQ ID NO: 7) | | | | | | | | | | | |
| PRV-P3 (SEQ ID NO: 8) | | | | | | | | | | | |
| PRV-F4 (SEQ ID NO: 9) | | - | - | - | - | - | - | - | - | - | - |
| PRV-R4 (SEQ ID NO: 10) | + | | | | | | | | | | |
| PRV-P3 (SEQ ID NO: 8) | | | | | | | | | | | |
| PRV-F5 (SEQ ID NO: 11) | + | - | - | - | - | - | - | - | - | - | - |
| PRV-R5 (SEQ ID NO: 12) | | | | | | | | | | | |
| PRV-P5 (SEQ ID NO: 13) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pPRV-L1 or pPRV-S3 plasmid (10¹, 10², 10³, 10⁴, 10⁵, 10⁶, 10⁷ copies). Real-time PCR assays were performed with 2 µl pPRV-L1 or pPRV-S3 plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pPRV-L1 or pPRV-S3 plasmid was calculated for the real-time PCR assays. At least 10² copies of pPRV-L1 or pPRV-S3 plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of PRV with high sensitivity and specificity.

### Example 2 Detection of Infectious Pancreatic Necrosis Virus (IPNV)

The VP5 and polyprotein genes of IPNV (GenBank accession No. KY548514) was inserted in a cloning vector to obtain pIPNV plasmid. The cloning vector can be, but is not limited to, pUC57 and pGEM-T.

### 1. Conventional PCR

The 50 µl PCR mixture for conventional PCR contains 10⁶ copies of pIPNV plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 4, indicating that each primer pair of the invention has correctly amplified the target sequence, whereas no target sequence has been amplified in the negative control (data not shown). The results demonstrate that the primer pairs of the invention can be used in conventional PCR amplification to detect the existence of IPNV.

**Table 4 The results of the conventional PCR**

| Forward Primer | Reverse Primer | Size of Target Sequence (bp) |
|---|---|---|
| IPNV-F1 (SEQ ID NO: 14) | IPNV-R1 (SEQ ID NO: 15) | 86 |
| IPNV-F2 (SEQ ID NO: 17) | IPNV-R2 (SEQ ID NO: 18) | 104 |
| IPNV-F3 (SEQ ID NO: 20) | IPNV-R3 (SEQ ID NO: 21) | 88 |
| IPNV-F4 (SEQ ID NO: 23) | IPNV-R4 (SEQ ID NO: 24) | 91 |

### 2. Convective PCR (cPCR)

The 50 µl PCR mixture, containing the mRNA (10², 10³, 10⁴, 10⁵, 10⁶ copies/µl respectively) of VP5 and polyprotein genes of IPNV or pIPNV plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 5, indicating that each combination of the primer pair and the probe of the invention has 100% correctly detected samples that contain 10² copies/µl of the mRNA of VP5 and polyprotein genes of IPNV or 10² copies of pIPNV plasmid. The sensitivity of each primer pair and probe is 10² copies/µl and 10² copies.

**Table 5 The cPCR sensitivity of each combination of primer pair and probe (n=8)**

| Combination of Primer Pair and Probe Positive Rate (%) | NC* | mRNA content of VP5 and polyprotein genes of IPNV (copies/µl) | | | | | pIPNV plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| IPNV-F 1 (SEQ ID NO: 14) | 0 | 100 | 100 | 100 | 100 | 100 | 100 | 100 | 100 | 100 | 100 |
| IPNV-R1 (SEQ ID NO: 15) | | | | | | | | | | | |
| IPNV-P1 (SEQ ID NO: 16) | | | | | | | | | | | |
| IPNV-F2 (SEQ ID NO: 17) | 0 | 100 | 100 | 100 | 100 | 100 | 100 | 100 | 100 | 100 | 100 |
| IPNV-R2 (SEQ ID NO: 18) | | | | | | | | | | | |
| IPNV-P2 (SEQ ID NO: 19) | | | | | | | | | | | |
| IPNV-F3 (SEQ ID NO: 20) | 0 | 100 | 100 | 100 | 100 | 100 | 100 | 100 | 100 | 100 | 100 |
| IPNV-R3 (SEQ ID NO: 21) | | | | | | | | | | | |
| IPNV-P3 (SEQ ID NO: 22) | | | | | | | | | | | |
| IPNV-F4 (SEQ ID NO: 23) | 0 | 100 | 100 | 100 | 100 | 100 | 100 | 100 | 100 | 100 | 100 |
| IPNV-R4 (SEQ ID NO: 24) | | | | | | | | | | | |
| IPNV-P4 (SEQ ID NO: 25) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 6, indicating that each combination of primer pair and probe of the invention has correctly detected samples that contain IPNV but not samples that contain PRV, ISAV, PMCV, SAV, *N. perurans* (indicated with AGD in Table 6), C. *Branchiomonas cysticola* (CBc), *D. lepeophtherii* (Des), SGPV, *P. salmonis* (indicated with SRS in Table 6), and *R. salmoninarum* (indicated with BKD in Table 6). The results demonstrate that all the combinations of the primer pairs and the probes of the invention is specific to IPNV.

**Table 6 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| IPNV-F1 (SEQ ID NO: 14) | - | + | - | - | - | - | - | - | - | - | - |
| IPNV-R1 (SEQ ID NO: 15) | | | | | | | | | | | |
| IPNV-P1 (SEQ ID NO: 16) | | | | | | | | | | | |
| IPNV-F2 (SEQ ID NO: 17) | - | + | - | - | - | - | - | - | - | - | - |
| IPNV-R2 (SEQ ID NO: 18) | | | | | | | | | | | |
| IPNV-P2 (SEQ ID NO: 19) | | | | | | | | | | | |
| IPNV-F3 (SEQ ID NO: 20) | - | | - | - | - | - | - | - | - | - | - |
| IPNV-R3 (SEQ ID NO: 21) | | + | | | | | | | | | |
| IPNV-P3 (SEQ ID NO: 22) | | | | | | | | | | | |
| IPNV-F4 (SEQ ID NO: 23) | - | + | - | - | - | - | - | - | - | - | - |
| IPNV-R4 (SEQ ID NO: 24) | | | | | | | | | | | |
| IPNV-P4 (SEQ ID NO: 25) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pIPNV plasmid (10¹, 10², 10³, 10⁴, 10⁵, 10⁶, 10⁷ copies). Real-time PCR assays were performed with 2 µl pIPNV plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pPRV-L1 plasmid was calculated for the real-time PCR assays. At least 10 copies of pIPNV plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of IPNV with high sensitivity and specificity.

### Example 3 Detection of Infectious salmon anemia virus (ISAV)

The ORF1 and ORF2 genes of ISAV (GenBank accession No. KX424589) was inserted in a cloning vector to obtain pISAV plasmid. The cloning vector can be, but is not limited to, pUC57 and pGEM-T.

### 1. Conventional PCR

The 50 µl PCR mixture for conventional PCR contains 10⁶ copies of pISAV plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 7, indicating that each primer pair of the invention has correctly amplified the target sequence, whereas no target sequence has been amplified in the negative control (data not shown). The results demonstrate that the primer pairs of the invention can be used in conventional PCR amplification to detect the existence of ISAV.

**Table 7 The results of the conventional PCR**

| Forward Primer | Reverse Primer | Size of Target Sequence (bp) |
|---|---|---|
| ISAV-F1 (SEQ ID NO: 26) | ISAV-R1 (SEQ ID NO: 27) | 87 |
| ISAV-F2 (SEQ ID NO: 29) | ISAV-R2 (SEQ ID NO: 30) | 83 |
| ISAV-F3 (SEQ ID NO: 32) | ISAV-R3 (SEQ ID NO: 33) | 77 |
| ISAV-F4 (SEQ ID NO: 35) | ISAV-R4 (SEQ ID NO: 36) | 83 |
| ISAV-F5 (SEQ ID NO: 37) | ISAV-R5 (SEQ ID NO: 38) | 96 |

### 2. Convective PCR (cPCR)

The 50 µl PCR mixture, containing the mRNA (10², 10³, 10⁴, 10⁵, 10⁶ copies/µl respectively) of ORF1 and ORF2 genes of ISAV or pISAV plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 8, indicating that each combination of the primer pair and the probe of the invention has 100% correctly detected samples that contain 10² copies/µl of the mRNA of ORF1 and ORF2 genes of ISAV or 10² copies of pISAV plasmid. The sensitivity of each primer pair and probe is 10² copies/µl and 10² copies.

**Table 8 The cPCR sensitivity of each combination of primer pair and probe (n=8)**

| Combination of Primer Pair and Probe Positive Rate (%) | NC* | mRNA content of ORF 1 and ORF2 genes of ISAV (copies/µl) | | | | | pISAV plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| ISAV-F1 (SEQ ID NO: 26) | 0 | 100 | 100 | 100 | 100 | 100 | 100 | 100 | 100 | 100 | 100 |
| ISAV-R1 (SEQ ID NO: 27) | | | | | | | | | | | |
| ISAV-P1 (SEQ ID NO: 28) | | | | | | | | | | | |
| ISAV-F2 (SEQ ID NO: 29) | 0 | 100 | 100 | 100 | 100 | 100 | 100 | 100 | 100 | 100 | 100 |
| ISAV-R2 (SEQ ID NO: 30) | | | | | | | | | | | |
| ISAV-P2 (SEQ ID NO: 31) | | | | | | | | | | | |
| ISAV-F3 (SEQ ID NO: 32) | 0 | 100 | 100 | 100 | 100 | 100 | 100 | 100 | 100 | 100 | 100 |
| ISAV-R3 (SEQ ID NO: 33) | | | | | | | | | | | |
| ISAV-P3 (SEQ ID NO: 34) | | | | | | | | | | | |
| ISAV-F4 (SEQ ID NO: 35) | 0 | 100 | 100 | 100 | 100 | 100 | 100 | 100 | 100 | 100 | 100 |
| ISAV-R4 (SEQ ID NO: 36) | | | | | | | | | | | |
| ISAV-P3 (SEQ ID NO: 34) | | | | | | | | | | | |
| ISAV-F5 (SEQ ID NO: 37) | 0 | 100 | 100 | 100 | 100 | 100 | 100 | 100 | 100 | 100 | 100 |
| ISAV-R5 (SEQ ID NO: 38) | | | | | | | | | | | |
| ISAV-P3 (SEQ ID NO: 34) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 9, indicating that each combination of primer pair and probe of the invention has correctly detected samples that contain ISAV but not samples that contain PRV, IPNV, PMCV, SAV, *N. perurans* (indicated with AGD in Table 9), C. *Branchiomonas cysticola* (CBc), *D. lepeophtherii* (Des), SGPV, *P. salmonis* (indicated with SRS in Table 9), and *R. salmoninarum* (indicated with BKD in Table 9). The results demonstrate that all the combinations of the primer pairs and the probes of the invention is specific to ISAV.

**Table 9 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| ISAV-F1 (SEQ ID NO: 26) | - | - | + | - | - | - | - | - | - | - | - |
| ISAV-R1 (SEQ ID NO: 27) | | | | | | | | | | | |
| ISAV-P1 (SEQ ID NO: 28) | | | | | | | | | | | |
| ISAV-F2 (SEQ ID NO: 29) | - | - | + | - | - | - | - | - | - | - | - |
| ISAV-R2 (SEQ ID NO: 30) | | | | | | | | | | | |
| ISAV-P2 (SEQ ID NO: 31) | | | | | | | | | | | |
| ISAV-F3 (SEQ ID NO: 32) | - | - | + | - | - | - | - | - | - | - | - |
| ISAV-R3 (SEQ ID NO: 33) | | | | | | | | | | | |
| ISAV-P3 (SEQ ID NO: 34) | | | | | | | | | | | |
| ISAV-F4 (SEQ ID NO: 35) | - | - | + | - | - | - | - | - | - | - | - |
| ISAV-R4 (SEQ ID NO: 36) | | | | | | | | | | | |
| ISAV-P3 (SEQ ID NO: 34) | | | | | | | | | | | |
| ISAV-F5 (SEQ ID NO: 37) | - | - | + | - | - | - | - | - | - | - | - |
| ISAV-R5 (SEQ ID NO: 38) | | | | | | | | | | | |
| ISAV-P3 (SEQ ID NO: 34) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pISAV plasmid (10¹, 10², 10³, 10⁴, 10⁵, 10⁶, 10⁷ copies). Real-time PCR assays were performed with 2 µl pISAV plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pISAV plasmid was calculated for the real-time PCR assays. At least 10² copies of pISAV plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of ISAV with high sensitivity and specificity.

### Example 4 Detection of Piscine Myocarditis Virus (PMCV)

A fragment of PMCV genome (GenBank accession No. HQ339954) was inserted in a cloning vector to obtain pPMCV plasmid. The cloning vector can be, but is not limited to, pUC57 and pGEM-T.

### 1. Conventional PCR

The 50 µl PCR mixture for conventional PCR contains 10⁶ copies of pPMCV plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 10, indicating that each primer pair of the invention has correctly amplified the target sequence, whereas no target sequence has been amplified in the negative control (data not shown). The results demonstrate that the primer pairs of the invention can be used in conventional PCR amplification to detect the existence of PMCV

**Table 10 The results of the conventional PCR**

| Forward Primer | Reverse Primer | Size of Target Sequence (bp) |
|---|---|---|
| PMCV-F1 (SEQ ID NO: 39) | PMCV-R1 (SEQ ID NO: 40) | 81 |
| PMCV-F2 (SEQ ID NO: 42) | PMCV-R2 (SEQ ID NO: 43) | 81 |
| PMCV-F3 (SEQ ID NO: 45) | PMCV-R3 (SEQ ID NO: 46) | 95 |
| PMCV-F4 (SEQ ID NO: 48) | PMCV-R4 (SEQ ID NO: 49) | 80 |
| PMCV-F5 (SEQ ID NO: 51) | PMCV-R4 (SEQ ID NO: 49) | 100 |
| PMCV-F6 (SEQ ID NO: 52) | PMCV-R4 (SEQ ID NO: 49) | 83 |

### 2. Convective PCR (cPCR)

The 50 µl PCR mixture, containing the mRNA (10², 10³, 10⁴, 10⁵, 10⁶ copies/µl respectively) of the PMCV genome fragment or pPMCV plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 11, indicating that each combination of the primer pair and the probe of the invention has 100% correctly detected samples that contain 10² copies/µl of the mRNA of PMCV genome or 10² copies of pPMCV plasmid. The sensitivity of each primer pair and probe is 10² copies/µl and 10² copies.

**Table 11 The cPCR sensitivity of each combination of primer pair and probe (n=8)**

| Combination of Primer Pair and Probe Positive Rate (%) | NC* | mRNA content of the PMCV genome fragment (copies/µl) | | | | | pPMCV plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| PMCV-F1 (SEQ ID NO: 39) | 0 | 100 | 100 | 100 | 100 | 100 | 100 | 100 | 100 | 100 | 100 |
| PMCV-R1 (SEQ ID NO: 40) | | | | | | | | | | | |
| PMCV-P1 (SEQ ID NO: 41) | | | | | | | | | | | |
| PMCV-F2 (SEQ ID NO: 42) | 0 | 100 | 100 | 100 | 100 | 100 | 100 | 100 | 100 | 100 | 100 |
| PMCV-R2 (SEQ ID NO: 43) | | | | | | | | | | | |
| PMCV-P2 (SEQ ID NO: 44) | | | | | | | | | | | |
| PMCV-F3 (SEQ ID NO: 45) | 0 | 100 | 100 | 100 | 100 | 100 | 100 | 100 | 100 | 100 | 100 |
| PMCV-R3 (SEQ ID NO: 46) | | | | | | | | | | | |
| PMCV-P3 (SEQ ID NO: 47) | | | | | | | | | | | |
| PMCV-F4 (SEQ ID NO: 48) | 0 | 100 | 100 | 100 | 100 | 100 | 100 | 100 | 100 | 100 | 100 |
| PMCV-R4 (SEQ ID NO: 49) | | | | | | | | | | | |
| PMCV-P4 (SEQ ID NO: 50) | | | | | | | | | | | |
| PMCV-F5 (SEQ ID NO: 51) | 0 | 100 | 100 | 100 | 100 | 100 | 100 | 100 | 100 | 100 | 100 |
| PMCV-R4 (SEQ ID NO: 49) | | | | | | | | | | | |
| PMCV-P4 (SEQ ID NO: 50) | | | | | | | | | | | |
| PMCV-F6 (SEQ ID NO: 52) | 0 | 100 | 100 | 100 | 100 | 100 | 100 | 100 | 100 | 100 | 100 |
| PMCV-R4 (SEQ ID NO: 49) | | | | | | | | | | | |
| PMCV-P4 (SEQ ID NO: 50) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 12, indicating that each combination of primer pair and probe of the invention has correctly detected samples that contain PMCV but not samples that contain PRV, IPNV, ISAV, SAV, *N. perurans* (indicated with AGD in Table 12), C. *Branchiomonas cysticola* (CBc), *D. lepeophtherii* (Des), SGPV, *P. salmonis* (indicated with SRS in Table 12), and *R. salmoninarum* (indicated with BKD in Table 12). The results demonstrate that all the combinations of the primer pairs and the probes of the invention is specific to PMCV.

**Table 12 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| PMCV-F1 (SEQ ID NO: 39) | - | - | - | + | - | - | - | - | - | - | - |
| PMCV-R1 (SEQ ID NO: 40) | | | | | | | | | | | |
| PMCV-P1 (SEQ ID NO: 41) | | | | | | | | | | | |
| PMCV-F2 (SEQ ID NO: 42) | - | - | - | + | - | - | - | - | - | - | - |
| PMCV-R2 (SEQ ID NO: 43) | | | | | | | | | | | |
| PMCV-P2 (SEQ ID NO: 44) | | | | | | | | | | | |
| PMCV-F3 (SEQ ID NO: 45) | - | - | - | + | - | - | - | - | - | - | - |
| PMCV-R3 (SEQ ID NO: 46) | | | | | | | | | | | |
| PMCV-P3 (SEQ ID NO: 47) | | | | | | | | | | | |
| PMCV-F4 (SEQ ID NO: 48) | - | - | - | + | - | - | - | - | - | - | - |
| PMCV-R4 (SEQ ID NO: 49) | | | | | | | | | | | |
| PMCV-P4 (SEQ ID NO: 50) | | | | | | | | | | | |
| PMCV-F5 (SEQ ID NO: 51) | - | - | - | + | - | - | - | - | - | - | - |
| PMCV-R4 (SEQ ID NO: 49) | | | | | | | | | | | |
| PMCV-P4 (SEQ ID NO: 50) | | | | | | | | | | | |
| PMCV-F6 (SEQ ID NO: 52) | - | - | - | + | - | - | - | - | - | - | - |
| PMCV-R4 (SEQ ID NO: 49) | | | | | | | | | | | |
| PMCV-P4 (SEQ ID NO: 50) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pPMCV plasmid (10¹, 10², 10³, 10⁴, 10⁵, 10⁶, 10⁷ copies). Real-time PCR assays were performed with 2 µl pPMCV plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pPMCV plasmid was calculated for the real-time PCR assays. At least 10² copies of pPMCV plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of PMCV with high sensitivity and specificity.

### Example 5 Detection of Salmonid Alphavirus (SAV)

A fragment of SAV genome (GenBank accession No. KC 122926) was inserted in a cloning vector to obtain pSAV plasmid. The cloning vector can be, but is not limited to, pUC57 and pGEM-T.

### 1. Conventional PCR

The 50 µl PCR mixture for conventional PCR contains 10⁶ copies of pSAV plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 13, indicating that each primer pair of the invention has correctly amplified the target sequence, whereas no target sequence has been amplified in the negative control (data not shown). The results demonstrate that the primer pairs of the invention can be used in conventional PCR amplification to detect the existence of SAV.

**Table 13 The results of the conventional PCR**

| Forward Primer | Reverse Primer | Size of Target Sequence (bp) |
|---|---|---|
| SAV-F1 (SEQ ID NO: 53) | SAV-R1 (SEQ ID NO: 54) | 87 |
| SAV-F2 (SEQ ID NO: 56) | SAV-R2 (SEQ ID NO: 57) | 77 |
| SAV-F3 (SEQ ID NO: 59) | SAV-R3 (SEQ ID NO: 60) | 110 |
| SAV-F4 (SEQ ID NO: 62) | SAV-R4 (SEQ ID NO: 63) | 84 |
| SAV-F5 (SEQ ID NO: 65) | SAV-R5 (SEQ ID NO: 66) | 83 |

### 2. Convective PCR (cPCR)

The 50 µl PCR mixture, containing the mRNA (10², 10³, 10⁴, 10⁵, 10⁶ copies/µl respectively) of the SAV genome fragment or pSAV plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 14, indicating that each combination of the primer pair and the probe of the invention has 100% correctly detected samples that contain 10² copies/µl of the mRNA of SAV genome or 10² copies of pSAV plasmid. The sensitivity of each primer pair and probe is 10² copies/µl and 10² copies.

**Table 14 The cPCR sensitivity of each combination of primer pair and probe (n=8)**

| Combination of Primer Pair and Probe Positive Rate (%) | NC* | mRNA content of the SAV genome fragment (copies/µl) | | | | | pSAV plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| SAV-F1 (SEQ ID NO: 53) | 0 | 100 | 100 | 100 | 100 | 100 | 100 | 100 | 100 | 100 | 100 |
| SAV-R1 (SEQ ID NO: 54) | | | | | | | | | | | |
| SAV-P1 (SEQ ID NO: 55) | | | | | | | | | | | |
| SAV-F2 (SEQ ID NO: 56) | 0 | 100 | 100 | 100 | 100 | 100 | 100 | 100 | 100 | 100 | 100 |
| SAV-R2 (SEQ ID NO: 57) | | | | | | | | | | | |
| SAV-P2 (SEQ ID NO: 58) | | | | | | | | | | | |
| SAV-F3 (SEQ ID NO: 59) | 0 | 100 | 100 | 100 | 100 | 100 | 100 | 100 | 100 | 100 | 100 |
| SAV-R3 (SEQ ID NO: 60) | | | | | | | | | | | |
| SAV-P3 (SEQ ID NO: 61) | | | | | | | | | | | |
| SAV-F4 (SEQ ID NO: 62) | 0 | 100 | 100 | 100 | 100 | 100 | 100 | 100 | 100 | 100 | 100 |
| SAV-R4 (SEQ ID NO: 63) | | | | | | | | | | | |
| SAV-P4 (SEQ ID NO: 64) | | | | | | | | | | | |
| SAV-F5 (SEQ ID NO: 65) | 0 | 100 | 100 | 100 | 100 | 100 | 100 | 100 | 100 | 100 | 100 |
| SAV-R5 (SEQ ID NO: 66) | | | | | | | | | | | |
| SAV-P5 (SEQ ID NO: 67) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 15, indicating that each combination of primer pair and probe of the invention has correctly detected samples that contain SAV but not samples that contain PRV, IPNV, ISAV, PMCV, *N. perurans* (indicated with AGD in Table 15), C. *Branchiomonas cysticola* (CBc), *D. lepeophtherii* (Des), SGPV, *P. salmonis* (indicated with SRS in Table 15), and *R. salmoninarum* (indicated with BKD in Table 15). The results demonstrate that all the combinations of the primer pairs and the probes of the invention is specific to SAV.

**Table 15 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| SAV-F1 (SEQ ID NO: 53) | - | - | - | - | + | - | - | - | - | - | - |
| SAV-R1 (SEQ ID NO: 54) | | | | | | | | | | | |
| SAV-P1 (SEQ ID NO: 55) | | | | | | | | | | | |
| SAV-F2 (SEQ ID NO: 56) | - | - | - | - | + | - | - | - | - | - | - |
| SAV-R2 (SEQ ID NO: 57) | | | | | | | | | | | |
| SAV-P2 (SEQ ID NO: 58) | | | | | | | | | | | |
| SAV-F3 (SEQ ID NO: 59) | - | - | - | - | + | - | - | - | - | - | - |
| SAV-R3 (SEQ ID NO: 60) | | | | | | | | | | | |
| SAV-P3 (SEQ ID NO: 61) | | | | | | | | | | | |
| SAV-F4 (SEQ ID NO: 62) | - | - | - | - | + | - | - | - | - | - | - |
| SAV-R4 (SEQ ID NO: 63) | | | | | | | | | | | |
| SAV-P4 (SEQ ID NO: 64) | | | | | | | | | | | |
| SAV-F5 (SEQ ID NO: 65) | - | - | - | - | + | - | - | - | - | - | - |
| SAV-R5 (SEQ ID NO: 66) | | | | | | | | | | | |
| SAV-P5 (SEQ ID NO: 67) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pSAV (10¹, 10², 10³, 10⁴, 10⁵, 10⁶, 10⁷ copies). Real-time PCR assays were performed with 2 µl pSAV plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pSAV plasmid was calculated for the real-time PCR assays. At least 10² copies of pSAV plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of SAV with high sensitivity and specificity.

### Example 6 Detection of Neoparamoeba perurans

The gene of 18S rRNA of *N. perurans* (GenBank accession No. KU985058) was inserted in a cloning vector to obtain pAGD plasmid. The cloning vector can be, but is not limited to, pUC57 and pGEM-T.

### 1. Conventional PCR

The 50 µl PCR mixture for conventional PCR contains 10⁶ copies of pAGD plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 16, indicating that each primer pair of the invention has correctly amplified the target sequence, whereas no target sequence has been amplified in the negative control (data not shown). The results demonstrate that the primer pairs of the invention can be used in conventional PCR amplification to detect the existence of *N. perurans.*

**Table 16 The results of the conventional PCR**

| Forward Primer | Reverse Primer | Size of Target Sequence (bp) |
|---|---|---|
| AGD-F1 (SEQ ID NO: 68) | AGD-R1 (SEQ ID NO: 69) | 111 |
| AGD-F2 (SEQ ID NO: 71) | AGD-R2 (SEQ ID NO: 72) | 95 |
| AGD-F3 (SEQ ID NO: 74) | AGD-R3 (SEQ ID NO: 75) | 89 |
| AGD-F4 (SEQ ID NO: 77) | AGD-R4 (SEQ ID NO: 78) | 70 |

### 2. Convective PCR (cPCR)

The 50 µl PCR mixture, containing the mRNA (10², 10³, 10⁴, 10⁵, 10⁶ copies/µl respectively) of 18S rRNA of *N. perurans* or pAGD plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 17, indicating that each combination of the primer pair and the probe of the invention has 100% correctly detected samples that contain 10² copies/µl of the mRNA of 18S rRNA of *N. perurans* or 10² copies of pAGD plasmid. The sensitivity of each primer pair and probe is 10² copies/µl and 10² copies.

**Table 17 The cPCR sensitivity of each combination of primer pair and probe (n=8)**

| Combination of Primer Pair and Probe Positive Rate (%) | NC* | mRNA content of 18S rRNA of *N. perurans* (copies/µl) | | | | | pAGD plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| AGD-F1 (SEQ ID NO: 68) | 0 | 100 | 100 | 100 | 100 | 100 | 100 | 100 | 100 | 100 | 100 |
| AGD-R1 (SEQ ID NO: 69) | | | | | | | | | | | |
| AGD-P1 (SEQ ID NO: 70) | | | | | | | | | | | |
| AGD-F2 (SEQ ID NO: 71) | 0 | 100 | 100 | 100 | 100 | 100 | 100 | 100 | 100 | 100 | 100 |
| AGD-R2 (SEQ ID NO: 72) | | | | | | | | | | | |
| AGD-P2 (SEQ ID NO: 73) | | | | | | | | | | | |
| AGD-F3 (SEQ ID NO: 74) | 0 | 100 | 100 | 100 | 100 | 100 | 100 | 100 | 100 | 100 | 100 |
| AGD-R3 (SEQ ID NO: 75) | | | | | | | | | | | |
| AGD-P3 (SEQ ID NO: 76) | | | | | | | | | | | |
| AGD-F4 (SEQ ID NO: 77) | 0 | 100 | 100 | 100 | 100 | 100 | 100 | 100 | 100 | 100 | 100 |
| AGD-R4 (SEQ ID NO: 78) | | | | | | | | | | | |
| AGD-P4 (SEQ ID NO: 79) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 18, indicating that each combination of primer pair and probe of the invention has correctly detected samples that contain *N. perurans* (indicated with AGD in Table 18) but not samples that contain PRV, IPNV, ISAV, PMCV, SAV, C. *Branchiomonas cysticola* (CBc), *D. lepeophtherii* (Des), SGPV, *P. salmonis* (indicated with SRS in Table 18), and *R. salmoninarum* (indicated with BKD in Table 18). The results demonstrate that all the combinations of the primer pairs and the probes of the invention is specific to *N. perurans.*

**Table 18 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| AGD-F1 (SEQ ID NO: 68) | - | - | - | - | - | + | - | - | - | - | - |
| AGD-R1 (SEQ ID NO: 69) | | | | | | | | | | | |
| AGD-P1 (SEQ ID NO: 70) | | | | | | | | | | | |
| AGD-F2 (SEQ ID NO: 71) | - | - | - | - | - | + | - | - | - | - | - |
| AGD-R2 (SEQ ID NO: 72) | | | | | | | | | | | |
| AGD-P2 (SEQ ID NO: 73) | | | | | | | | | | | |
| AGD-F3 (SEQ ID NO: 74) | - | - | - | - | - | + | - | - | - | - | - |
| AGD-R3 (SEQ ID NO: 75) | | | | | | | | | | | |
| AGD-P3 (SEQ ID NO: 76) | | | | | | | | | | | |
| AGD-F4 (SEQ ID NO: 77) | - | - | - | - | - | + | - | - | - | - | - |
| AGD-R4 (SEQ ID NO: 78) | | | | | | | | | | | |
| AGD-P4 (SEQ ID NO: 79) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pAGD plasmid (10¹, 10², 10³, 10⁴, 10⁵, 10⁶, 10⁷ copies). Real-time PCR assays were performed with 2 µl pAGD plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pAGD plasmid was calculated for the real-time PCR assays. At least 10² copies of pAGD plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of *N. perurans* with high sensitivity and specificity.

### Example 7 Detection of Candidatus Branchiomonas cysticola (CBc)

The gene of 16S rRNA of CBc (GenBank accession No. JQ723599) was inserted in a cloning vector to obtain pCBc plasmid. The cloning vector can be, but is not limited to, pUC57 and pGEM-T.

### 1. Conventional PCR

The 50 µl PCR mixture for conventional PCR contains 10⁶ copies of pCBc plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 19, indicating that each primer pair of the invention has correctly amplified the target sequence, whereas no target sequence has been amplified in the negative control (data not shown). The results demonstrate that the primer pairs of the invention can be used in conventional PCR amplification to detect the existence of *Ca. B. cysticola* (CBc).

**Table 19 The results of the conventional PCR**

| Forward Primer | Reverse Primer | Size of Target Sequence (bp) |
|---|---|---|
| CBc-F1 (SEQ ID NO: 80) | CBc-R1 (SEQ ID NO: 81) | 98 |
| CBc-F2 (SEQ ID NO: 83) | CBc-R2 (SEQ ID NO: 84) | 70 |

### 2. Convective PCR (cPCR)

The 50 µl PCR mixture, containing the mRNA (10², 10³, 10⁴, 10⁵, 10⁶ copies/µl respectively) of 16S rRNA of CBc or pCBc plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 20, indicating that each combination of the primer pair and the probe of the invention has 100% correctly detected samples that contain 10² copies/µl of the mRNA of 16S rRNA of CBc or 10² copies of pCBc plasmid. The sensitivity of each primer pair and probe is 10² copies/µl and 10² copies.

**Table 20 The cPCR sensitivity of each combination of primer pair and probe (n=8)**

| Combination of Primer Pair and Probe Positive Rate (%) | NC* | mRNA content of 16S rRNA of CBc (copies/µl) | | | | | pCBc plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| CBc-F1 (SEQ ID NO: 80) | 0 | 100 | 100 | 100 | 100 | 100 | 100 | 100 | 100 | 100 | 100 |
| CBc-R1 (SEQ ID NO: 81) | | | | | | | | | | | |
| CBc-F2 (SEQ ID NO: 83) | 0 | 100 | 100 | 100 | 100 | 100 | 100 | 100 | 100 | 100 | 100 |
| CBc-R2 (SEQ ID NO: 84) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 21, indicating that each combination of primer pair and probe of the invention has correctly detected samples that contain *C*. *Branchiomonas cysticola* (CBc)but not samples that contain PRV, IPNV, ISAV, PMCV, SAV, *N. perurans* (indicated with AGD in Table 21), *D. lepeophtherii* (Des), SGPV, *P. salmonis* (indicated with SRS in Table 21), and *R. salmoninarum* (indicated with BKD in Table 21). The results demonstrate that all the combinations of the primer pairs and the probes of the invention is specific to CBc.

**Table 21 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| CBc-F1 (SEQ ID NO: 80) | - | - | - | - | - | - | + | - | - | - | - |
| CBc-R1 (SEQ ID NO: 81) | | | | | | | | | | | |
| CBc-F2 (SEQ ID NO: 83) | - | - | - | - | - | - | + | - | - | - | - |
| CBc-R2 (SEQ ID NO: 84) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pCBc plasmid (10¹, 10², 10³, 10⁴, 10⁵, 10⁶, 10⁷ copies). Real-time PCR assays were performed with 2 µl pCBc plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pCBc plasmid was calculated for the real-time PCR assays. At least 10² copies of pCBc plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of *C*. *Branchiomonas cysticola* (CBc) with high sensitivity and specificity.

### Example 8 Detection of Desmozoon lepeophtherii (Des)

The gene of 16S rRNA of *D. lepeophtherii* (Des) (GenBank accession No. AJ431366) was inserted in a cloning vector to obtain pDes plasmid. The cloning vector can be, but is not limited to, pUC57 and pGEM-T.

### 1. Conventional PCR

The 50 µl PCR mixture for conventional PCR contains 10⁶ copies of pDes plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 22, indicating that each primer pair of the invention has correctly amplified the target sequence, whereas no target sequence has been amplified in the negative control (data not shown). The results demonstrate that the primer pairs of the invention can be used in conventional PCR amplification to detect the existence of *D. lepeophtherii* (Des).

**Table 22 The results of the conventional PCR**

| Forward Primer | Reverse Primer | Size of Target Sequence (bp) |
|---|---|---|
| Des-F1 (SEQ ID NO: 86) | Des-R1 (SEQ ID NO: 87) | 92 |
| Des-F2 (SEQ ID NO: 89) | Des-R2 (SEQ ID NO: 90) | 82 |
| Des-F3 (SEQ ID NO: 92) | Des-R3 (SEQ ID NO: 93) | 91 |
| Des-F4 (SEQ ID NO: 95) | Des-R4 (SEQ ID NO: 97) | 89 |
| Des-F5 (SEQ ID NO: 96) | Des-R4 (SEQ ID NO: 97) | 87 |

### 2. Convective PCR (cPCR)

The 50 µl PCR mixture, containing the mRNA (10², 10³, 10⁴, 10⁵, 10⁶ copies/µl respectively) of 16S rRNA of *D. lepeophtherii* (Des) or pDes plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 23, indicating that each combination of the primer pair and the probe of the invention has 100% correctly detected samples that contain 10² copies/µl of the mRNA of 16S rRNA of *D. lepeophtherii* (Des) or 10² copies of pDes plasmid. The sensitivity of each primer pair and probe is 10² copies/µl and 10² copies.

**Table 23 The cPCR sensitivity of each combination of primer pair and probe (n=8)**

| Combination of Primer Pair and Probe Positive Rate (%) | NC* | mRNA content of 16S rRNA of Des (copies/µl) | | | | | pDes plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| Des-F1 (SEQ ID NO: 86) | 0 | 100 | 100 | 100 | 100 | 100 | 100 | 100 | 100 | 100 | 100 |
| Des-R1 (SEQ ID NO: 87) | | | | | | | | | | | |
| Des-P1 (SEQ ID NO: 88) | | | | | | | | | | | |
| Des-F2 (SEQ ID NO: 89) | 0 | 100 | 100 | 100 | 100 | 100 | 100 | 100 | 100 | 100 | 100 |
| Des-R2 (SEQ ID NO: 90) | | | | | | | | | | | |
| Des-P2 (SEQ ID NO: 91) | | | | | | | | | | | |
| Des-F3 (SEQ ID NO: 92) | 0 | 100 | 100 | 100 | 100 | 100 | 100 | 100 | 100 | 100 | 100 |
| Des-R3 (SEQ ID NO: 93) | | | | | | | | | | | |
| Des-P3 (SEQ ID NO: 94) | | | | | | | | | | | |
| Des-F4 (SEQ ID NO: 95) | 0 | 100 | 100 | 100 | 100 | 100 | 100 | 100 | 100 | 100 | 100 |
| Des-R4 (SEQ ID NO: 97) | | | | | | | | | | | |
| Des-P4 (SEQ ID NO: 98) | | | | | | | | | | | |
| Des-F5 (SEQ ID NO: 96) | 0 | 100 | 100 | 100 | 100 | 100 | 100 | 100 | 100 | 100 | 100 |
| Des-R4 (SEQ ID NO: 97) | | | | | | | | | | | |
| Des-P4 (SEQ ID NO: 98) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 24, indicating that each combination of primer pair and probe of the invention has correctly detected samples that contain *D. lepeophtherii* (Des) but not samples that contain PRV, IPNV, ISAV, PMCV, SAV, *N. perurans* (indicated with AGD in Table 24), *C. Branchiomonas cysticola* (CBc), SGPV, *P. salmonis* (indicated with SRS in Table 24), and *R. salmoninarum* (indicated with BKD in Table 24). The results demonstrate that all the combinations of the primer pairs and the probes of the invention is specific to *D. lepeophtherii* (Des).

**Table 24 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| Des-F1 (SEQ ID NO: 86) | - | - | - | - | - | - | - | + | - | - | - |
| Des-R1 (SEQ ID NO: 87) | | | | | | | | | | | |
| Des-P1 (SEQ ID NO: 88) | | | | | | | | | | | |
| Des-F2 (SEQ ID NO: 89) | - | - | - | - | - | - | - | + | - | - | - |
| Des-R2 (SEQ ID NO: 90) | | | | | | | | | | | |
| Des-P2 (SEQ ID NO: 91) | | | | | | | | | | | |
| Des-F3 (SEQ ID NO: 92) | - | - | - | - | - | - | - | + | - | - | - |
| Des-R3 (SEQ ID NO: 93) | | | | | | | | | | | |
| Des-P3 (SEQ ID NO: 94) | | | | | | | | | | | |
| Des-F4 (SEQ ID NO: 95) | - | - | - | - | - | - | - | + | - | - | - |
| Des-R4 (SEQ ID NO: 97) | | | | | | | | | | | |
| Des-P4 (SEQ ID NO: 98) | | | | | | | | | | | |
| Des-F5 (SEQ ID NO: 96) | - | - | - | - | - | - | - | + | - | - | - |
| Des-R4 (SEQ ID NO: 97) | | | | | | | | | | | |
| Des-P4 (SEQ ID NO: 98) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pDes plasmid (10¹, 10², 10³, 10⁴, 10⁵, 10⁶, 10⁷ copies). Real-time PCR assays were performed with 2 µl pDes plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pDes plasmid was calculated for the real-time PCR assays. At least 10² copies of pDes plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of *D. lepeophtherii* (Des) with high sensitivity and specificity.

### Example 9 Detection of Salmon gill poxvirus (SGPV)

A fragment of SGPV genome (GenBank accession No. KT159937) was inserted in a cloning vector to obtain pSGPV plasmid. The cloning vector can be, but is not limited to, pUC57 and pGEM-T.

### 1. Conventional PCR

The 50 µl PCR mixture for conventional PCR contains 10⁶ copies of pSGPV plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 25, indicating that each primer pair of the invention has correctly amplified the target sequence, whereas no target sequence has been amplified in the negative control (data not shown). The results demonstrate that the primer pairs of the invention can be used in conventional PCR amplification to detect the existence of SGPV.

**Table 25 The results of the conventional PCR**

| Forward Primer | Reverse Primer | Size of Target Sequence (bp) |
|---|---|---|
| SGPV-F1 (SEQ ID NO: 99) | SGPV-R1 (SEQ ID NO: 100) | 84 |
| SGPV-F2 (SEQ ID NO: 102) | SGPV-R2 (SEQ ID NO: 103) | 74 |
| SGPV-F3 (SEQ ID NO: 105) | SGPV-R3 (SEQ ID NO: 106) | 82 |
| SGPV-F4 (SEQ ID NO: 108) | SGPV-R4 (SEQ ID NO: 109) | 106 |

### 2. Convective PCR (cPCR)

The 50 µl PCR mixture, containing the mRNA (10², 10³, 10⁴, 10⁵, 10⁶ copies/µl respectively) of the SGPV genome fragment or pSGPV plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 26, indicating that each combination of the primer pair and the probe of the invention has 100% correctly detected samples that contain 10² copies/µl of the mRNA of the SGPV genome fragment or 10² copies of pSGPV plasmid. The sensitivity of each primer pair and probe is 10² copies/µl and 10² copies.

**Table 26 The cPCR sensitivity of each combination of primer pair and probe (n=8)**

| Combination of Primer Pair and Probe Positive Rate (%) | NC* | mRNA content of the SGPV genome fragment (copies/µl) | | | | | pSGPV plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| SGPV-F1 (SEQ ID NO: 99) | 0 | 100 | 100 | 100 | 100 | 100 | 100 | 100 | 100 | 100 | 100 |
| SGPV-R1 (SEQ ID NO: 100) | | | | | | | | | | | |
| SGPV-P1 (SEQ ID NO: 101) | | | | | | | | | | | |
| SGPV-F2 (SEQ ID NO: 102) | 0 | 100 | 100 | 100 | 100 | 100 | 100 | 100 | 100 | 100 | 100 |
| SGPV-R2 (SEQ ID NO: 103) | | | | | | | | | | | |
| SGPV-P2 (SEQ ID NO: 104) | | | | | | | | | | | |
| SGPV-F3 (SEQ ID NO: 105) | 0 | 100 | 100 | 100 | 100 | 100 | 100 | 100 | 100 | 100 | 100 |
| SGPV-R3 (SEQ ID NO: 106) | | | | | | | | | | | |
| SGPV-P3 (SEQ ID NO: 107) | | | | | | | | | | | |
| SGPV-F4 (SEQ ID NO: 108) | 0 | 100 | 100 | 100 | 100 | 100 | 100 | 100 | 100 | 100 | 100 |
| SGPV-R4 (SEQ ID NO: 109) | | | | | | | | | | | |
| SGPV-P4 (SEQ ID NO: 110) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 27, indicating that each combination of primer pair and probe of the invention has correctly detected samples that contain SGPV but not samples that contain PRV, IPNV, ISAV, PMCV, SAV, *N. perurans* (indicated with AGD in Table 27), *C. Branchiomonas cysticola* (CBc), *D. lepeophtherii* (Des), *P. salmonis* (indicated with SRS in Table 27), and *R. salmoninarum* (indicated with BKD in Table 27). The results demonstrate that all the combinations of the primer pairs and the probes of the invention is specific to SGPV.

**Table 27 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| SGPV-F1 (SEQ ID NO: 99) | - | - | - | - | - | - | - | - | + | - | - |
| SGPV-R1 (SEQ ID NO: 100) | | | | | | | | | | | |
| SGPV-P1 (SEQ ID NO: 101) | | | | | | | | | | | |
| SGPV-F2 (SEQ ID NO: 102) | - | - | - | - | - | - | - | - | + | - | - |
| SGPV-R2 (SEQ ID NO: 103) | | | | | | | | | | | |
| SGPV-P2 (SEQ ID NO: 104) | | | | | | | | | | | |
| SGPV-F3 (SEQ ID NO: 105) | - | - | - | - | - | - | - | - | + | - | - |
| SGPV-R3 (SEQ ID NO: 106) | | | | | | | | | | | |
| SGPV-P3 (SEQ ID NO: 107) | | | | | | | | | | | |
| SGPV-F4 (SEQ ID NO: 108) | - | - | - | - | - | - | - | - | + | - | - |
| SGPV-R4 (SEQ ID NO: 109) | | | | | | | | | | | |
| SGPV-P4 (SEQ ID NO: 110) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pSGPV plasmid (10¹, 10², 10³, 10⁴, 10⁵, 10⁶, 10⁷ copies). Real-time PCR assays were performed with 2 µl pSGPV plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pSGPV plasmid was calculated for the real-time PCR assays. At least 10² copies of pSGPV plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of SGPV with high sensitivity and specificity.

### Example 10 Detection of Piscirickettsia salmonis

The gene of 16S rRNA of *P. salmonis* (GenBank accession No. AY498634) was inserted in a cloning vector to obtain pSRS plasmid. The cloning vector can be, but is not limited to, pUC57 and pGEM-T.

### 1. Conventional PCR

The 50 µl PCR mixture for conventional PCR contains 10⁶ copies of pSRS plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 28, indicating that each primer pair of the invention has correctly amplified the target sequence, whereas no target sequence has been amplified in the negative control (data not shown). The results demonstrate that the primer pairs of the invention can be used in conventional PCR amplification to detect the existence of *P. salmonis.*

**Table 28 The results of the conventional PCR**

| Forward Primer | Reverse Primer | Size of Target Sequence (bp) |
|---|---|---|
| SRS-F1 (SEQ ID NO: 111) | SRS-R1 (SEQ ID NO: 112) | 151 |
| SRS-F2 (SEQ ID NO: 114) | SRS-R2 (SEQ ID NO: 115) | 80 |
| SRS-F3 (SEQ ID NO: 116) | SRS-R3 (SEQ ID NO: 117) | 54 |
| SRS-F4 (SEQ ID NO: 119) | SRS-R4 (SEQ ID NO: 120) | 56 |
| SRS-F5 (SEQ ID NO: 122) | SRS-R5 (SEQ ID NO: 123) | 58 |

### 2. Convective PCR (cPCR)

The 50 µl PCR mixture, containing the mRNA (10², 10³, 10⁴, 10⁵, 10⁶ copies/µl respectively) of 16S rRNA of *P. salmonis* or pSRS plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 29, indicating that each combination of the primer pair and the probe of the invention has 100% correctly detected samples that contain 10² copies/µl of the mRNA of 16S rRNA of *P. salmonis* or 10² copies of pSRS plasmid. The sensitivity of each primer pair and probe is 10² copies/µl and 10² copies.

**Table 29 The cPCR sensitivity of each combination of primer pair and probe (n=8)**

| Combination of Primer Pair and Probe Positive Rate (%) | NC* | mRNA content of 16S rRNA of *P. salmonis* (copies/µl) | | | | | pSRS plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| SRS-F1 (SEQ ID NO: 111) | 0 | 100 | 100 | 100 | 100 | 100 | 100 | 100 | 100 | 100 | 100 |
| SRS-R1 (SEQ ID NO: 112) | | | | | | | | | | | |
| SRS-P1 (SEQ ID NO: 113) | | | | | | | | | | | |
| SRS-F2 (SEQ ID NO: 114) | 0 | 100 | 100 | 100 | 100 | 100 | 100 | 100 | 100 | 100 | 100 |
| SRS-R2 (SEQ ID NO: 115) | | | | | | | | | | | |
| SRS-P1 (SEQ ID NO: 113) | | | | | | | | | | | |
| SRS-F3 (SEQ ID NO: 116) | 0 | 100 | 100 | 100 | 100 | 100 | 100 | 100 | 100 | 100 | 100 |
| SRS-R3 (SEQ ID NO: 117) | | | | | | | | | | | |
| SRS-P3 (SEQ ID NO: 118) | | | | | | | | | | | |
| SRS-F4 (SEQ ID NO: 119) | 0 | 100 | 100 | 100 | 100 | 100 | 100 | 100 | 100 | 100 | 100 |
| SRS-R4 (SEQ ID NO: 120) | | | | | | | | | | | |
| SRS-P4 (SEQ ID NO: 121) | | | | | | | | | | | |
| SRS-F5 (SEQ ID NO: 122) | 0 | 100 | 100 | 100 | 100 | 100 | 100 | 100 | 100 | 100 | 100 |
| SRS-R5 (SEQ ID NO: 123) | | | | | | | | | | | |
| SRS-P5 (SEQ ID NO: 124) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 30, indicating that each combination of primer pair and probe of the invention has correctly detected samples that contain *P. salmonis* (indicated with SRS in Table 30) but not samples that contain PRV, IPNV, ISAV, PMCV, SAV, *N. perurans* (indicated with AGD in Table 30), *C. Branchiomonas cysticola* (CBc), *D. lepeophtherii* (Des), SGPV, and *R. salmoninarum* (indicated with BKD in Table 30). The results demonstrate that all the combinations of the primer pairs and the probes of the invention is specific to *P. salmonis.*

**Table 30 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| SRS-F1 (SEQ ID NO: 111) | - | - | - | - | - | - | - | - | - | + | - |
| SRS-R1 (SEQ ID NO: 112) | | | | | | | | | | | |
| SRS-P1 (SEQ ID NO: 113) | | | | | | | | | | | |
| SRS-F2 (SEQ ID NO: 114) | - | - | - | - | - | - | - | - | - | + | - |
| SRS-R2 (SEQ ID NO: 115) | | | | | | | | | | | |
| SRS-P1 (SEQ ID NO: 113) | | | | | | | | | | | |
| SRS-F3 (SEQ ID NO: 116) | - | - | - | - | - | - | - | - | - | + | - |
| SRS-R3 (SEQ ID NO: 117) | | | | | | | | | | | |
| SRS-P3 (SEQ ID NO: 118) | | | | | | | | | | | |
| SRS-F4 (SEQ ID NO: 119) | - | - | - | - | - | - | - | - | - | + | - |
| SRS-R4 (SEQ ID NO: 120) | | | | | | | | | | | |
| SRS-P4 (SEQ ID NO: 121) | | | | | | | | | | | |
| SRS-F5 (SEQ ID NO: 122) | - | - | - | - | - | - | - | - | - | + | - |
| SRS-R5 (SEQ ID NO: 123) | | | | | | | | | | | |
| SRS-P5 (SEQ ID NO: 124) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pSRS plasmid (10¹, 10², 10³, 10⁴, 10⁵, 10⁶, 10⁷ copies). Real-time PCR assays were performed with 2 µl pSRS plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pSRS plasmid was calculated for the real-time PCR assays. At least 10² copies of pSRS plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of *P. salmonis* with high sensitivity and specificity.

### Example 11 Detection of Renibacterium salmoninarum

The gene of 57KD extracellular protein precursor (ECP) of *R. salmoninarum* (GenBank accession No. Z12174) was inserted in a cloning vector to obtain pBKD plasmid. The cloning vector can be, but is not limited to, pUC57 and pGEM-T.

### 1. Conventional PCR

The 50 µl PCR mixture for conventional PCR contains 10⁶ copies of pBKD plasmid, 0.01-2 µM forward primer, 0.01-2 µM reverse primer, 0.2 µM dNTP, and 1.25U Taq DNA polymerase. The amplification was performed in a thermal cycler (such as, but not limited to, PC818, Astec Co. Ltd., Japan) and consisted of one initial cycle of denaturation for 3 minutes at 94°C and 35 cycles of 30 seconds at 94°C, 30 seconds at 60°C, and 30 seconds of extension at 72°C. The amplicons were analyzed subsequently on a 15% polyacrylamide gel in TAE buffer and visualized by ethidium bromide staining.

The results of the conventional PCR are shown in Table 31, indicating that each primer pair of the invention has correctly amplified the target sequence, whereas no target sequence has been amplified in the negative control (data not shown). The results demonstrate that the primer pairs of the invention can be used in conventional PCR amplification to detect the existence of *R. salmoninarum.*

**Table 31 The results of the conventional PCR**

| Forward Primer | Reverse Primer | Size of Target Sequence (bp) |
|---|---|---|
| BKD-F1 (SEQ ID NO: 125) | BKD-R1 (SEQ ID NO: 128) | 72 |
| BKD-F2 (SEQ ID NO: 126) | BKD-R1 (SEQ ID NO: 128) | 85 |
| BKD-F3 (SEQ ID NO: 127) | BKD-R1 (SEQ ID NO: 128) | 70 |
| BKD-OF1 (SEQ ID NO: 130) | BKD-OR1 (SEQ ID NO: 131) | 65 |
| BKD-OF2 (SEQ ID NO: 133) | BKD-OR2 (SEQ ID NO: 134) | 125 |
| BKD-OF3 (SEQ ID NO: 136) | BKD-OR3 (SEQ ID NO: 137) | 133 |

### 2. Convective PCR (cPCR)

The 50 µl PCR mixture, containing the mRNA (10², 10³, 10⁴, 10⁵, 10⁶ copies/µl respectively) of the gene of 57KD ECP of *R. salmoninarum* or pBKD plasmid (10², 10³, 10⁴, 10⁵, 10⁶ copies respectively), 0.01-2 µM forward primer, 0.01-2 µM reverse primer, 0.01-2 µM probe (a fluorescer molecule, FAM, and a quencher molecule, BHQ1, attach to the 5' end and 3' end of each probe sequence, respectively), 0.2 µM dNTP, 1X qPCR buffer, 1-5 units of *Taq* DNA polymerase, and 1-5 units of reverse transcriptase, was added in a reaction tube and incubated in a cPCR machine for a designated period of time (about 30 to 45 minutes). The fluorescence of FAM in each sample was detected by the cPCR machine. Repeat the cPCR test 8 times (n = 8) to evaluate the sensitivity of each primer pair and probe.

The results of the sensitivity test are shown in Table 32, indicating that each combination of the primer pair and the probe of the invention has 100% correctly detected samples that contain 10² copies/µl of the mRNA of the gene of 57KD ECP of *R. salmoninarum* or 10² copies of pBKD plasmid. The sensitivity of each primer pair and probe is 10² copies/µl and 10² copies.

**Table 32 The cPCR sensitivity of each combination of primer pair and probe (n=8)**

| Combination of Primer Pair and Probe Positive Rate (%) | NC* | mRNA content of the gene of 57KD ECP of *R. salmoninarum* (copies/µl) | | | | | pBKD plasmid content (copies) | | | | |
|---|---|---|---|---|---|---|---|---|---|---|---|
| | | 10² | 10³ | 10⁴ | 10⁵ | 10⁶ | 10² | 10³ | 10⁴ | 10⁵ | 10⁶ |
| BKD-F 1 (SEQ ID NO: 125) | 0 | 100 | 100 | 100 | 100 | 100 | 100 | 100 | 100 | 100 | 100 |
| BKD-R1 (SEQ ID NO: 128) | | | | | | | | | | | |
| BKD-P1 (SEQ ID NO: 129) | | | | | | | | | | | |
| BKD-F2 (SEQ ID NO: 126) | 0 | 100 | 100 | 100 | 100 | 100 | 100 | 100 | 100 | 100 | 100 |
| BKD-R1 (SEQ ID NO: 128) | | | | | | | | | | | |
| BKD-P1 (SEQ ID NO: 129) | | | | | | | | | | | |
| BKD-F3 (SEQ ID NO: 127) | 0 | 100 | 100 | 100 | 100 | 100 | 100 | 100 | 100 | 100 | 100 |
| BKD-R1 (SEQ ID NO: 128) | | | | | | | | | | | |
| BKD-P1 (SEQ ID NO: 129) | | | | | | | | | | | |
| BKD-OF1 (SEQ ID NO: 130) | 0 | 100 | 100 | 100 | 100 | 100 | 100 | 100 | 100 | 100 | 100 |
| BKD-OR1 (SEQ ID NO: 131) | | | | | | | | | | | |
| BKD-OP1 (SEQ ID NO: 132) | | | | | | | | | | | |
| BKD-OF2 (SEQ ID NO: 133) | 0 | 100 | 100 | 100 | 100 | 100 | 100 | 100 | 100 | 100 | 100 |
| BKD-OR2 (SEQ ID NO: 134) | | | | | | | | | | | |
| BKD-OP2 (SEQ ID NO: 135) | | | | | | | | | | | |
| BKD-OF3 (SEQ ID NO: 136) | 0 | 100 | 100 | 100 | 100 | 100 | 100 | 100 | 100 | 100 | 100 |
| BKD-OR3 (SEQ ID NO: 137) | | | | | | | | | | | |
| BKD-OP3 (SEQ ID NO: 138) | | | | | | | | | | | |

| | | | | | | | | | | | |
|---|---|---|---|---|---|---|---|---|---|---|---|
| * NC: negative control. | | | | | | | | | | | |

In addition, specificity of each combination of the primer pair and the probe of the invention was evaluated using plasmids containing different fish pathogen genes (10⁶ copies/µl) as the template. Method of cPCR is as described above. The results of the specificity test are shown in Table 33, indicating that each combination of primer pair and probe of the invention has correctly detected samples that contain *R. salmoninarum* (indicated with BKD in Table 33) but not samples that contain PRV, IPNV, ISAV, PMCV, SAV, *N. perurans* (indicated with AGD in Table 33), *C. Branchiomonas cysticola* (CBc), *D. lepeophtherii* (Des), SGPV, and *P. salmonis* (indicated with SRS in Table 33). The results demonstrate that all the combinations of the primer pairs and the probes of the invention is specific to *R. salmoninarum.*

**Table 33 The cPCR specificity of each combination of primer pair and probe**

| Combination of Primer Pair and Probe | PRV | IPNV | ISAV | PMCV | SAV | AGD | CBc | Des | SGPV | SRS | BKD |
|---|---|---|---|---|---|---|---|---|---|---|---|
| BKD-F1 (SEQ ID NO: 125) | - | - | - | - | - | - | - | - | - | - | + |
| BKD-R1 (SEQ ID NO: 128) | | | | | | | | | | | |
| BKD-P1 (SEQ ID NO: 129) | | | | | | | | | | | |
| BKD-F2 (SEQ ID NO: 126) | - | - | - | - | - | - | - | - | - | - | + |
| BKD-R1 (SEQ ID NO: 128) | | | | | | | | | | | |
| BKD-P1 (SEQ ID NO: 129) | | | | | | | | | | | |
| BKD-F3 (SEQ ID NO: 127) | - | - | - | - | - | - | - | - | - | - | + |
| BKD-R1 (SEQ ID NO: 128) | | | | | | | | | | | |
| BKD-P1 (SEQ ID NO: 129) | | | | | | | | | | | |
| BKD-OF1 (SEQ ID NO: 130) | - | - | - | - | - | - | - | - | - | - | + |
| BKD-OR1 (SEQ ID NO: 131) | | | | | | | | | | | |
| BKD-OP1 (SEQ ID NO: 132) | | | | | | | | | | | |
| BKD-OF2 (SEQ ID NO: 133) | - | - | - | - | - | - | - | - | - | - | + |
| BKD-OR2 (SEQ ID NO: 134) | | | | | | | | | | | |
| BKD-OP2 (SEQ ID NO: 135) | | | | | | | | | | | |
| BKD-OF3 (SEQ ID NO: 136) | - | - | - | - | - | - | - | - | - | - | + |
| BKD-OR3 (SEQ ID NO: 137) | | | | | | | | | | | |
| BKD-OP3 (SEQ ID NO: 138) | | | | | | | | | | | |

| | | | | | | | | | | | |
|---|---|---|---|---|---|---|---|---|---|---|---|
| "+" indicates fluorescent signal was detected in a sample; "-" indicates no fluorescent signal was detected in a sample. | | | | | | | | | | | |

### 3. Real-time PCR (qPCR)

Real-time PCR assay were carried out in a real time PCR machine (such as, but not limited to ABI StepOnePlus™; Applied BioSystem, Life Technologies, CA, USA) using diluted pBKD plasmid (10¹, 10², 10³, 10⁴, 10⁵, 10⁶, 10⁷ copies). Real-time PCR assays were performed with 2 µl pBKD plasmid, 0.01-2 µM forward primer, 0.01-2 µM reverse primer, and 0.01-2 µM probe (a fluorescer molecule, FAM, and a quencher molecule, BHQ1, attach to the 5' end and 3' end of each probe sequence, respectively) in a total volume of 20 µl by using a commercial RT-PCR Kit (such as, but not limited to, OneStep PrimeScript™ RT-PCR Kit; Takara Bio Inc., Japan). The program included an incubation period at 42°C 5 minutes, 94°C for 10 seconds, and 40 cycles of 94°C for 10 seconds and 60°C for 30 minutes. Fluorescence measurements were recorded at the 60°C step. The standard curve for the serial dilutions (10-fold) of the pBKD plasmid was calculated for the real-time PCR assays. At least 10² copies of pBKD plasmid can be detected. The R² value of the standard curve of each combination of primer pair and probe is above 0.99, indicating the primers and the probe of the invention can be used in real-time PCR to produce reliable outcomes.

The above results demonstrate that the primers and the probes of the invention can be used in cPCR amplification to detect the existence of *R. salmoninarum* with high sensitivity and specificity.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A pair of oligonucleotides for detecting pathogens in fish, comprising a first primer and a second primer, and the pathogens being selected from the group consisting of piscine reovirus (PRV), infectious pancreatic necrosis virus (IPNV), infectious salmon anemia virus (ISAV), piscine myocarditis virus (PMCV), salmonid alphavirus (SAV), *Neoparamoeba perurans, Candidatus Branchiomonas cysticola* (CBc), *Desmozoon lepeophtherii* (Des), salmon gill poxvirus (SGPV), *Piscirickettsia salmonis,* and *Renibacterium salmoninarum;*
wherein when the pathogen is piscine reovirus (PRV), a sequence combination of the first primer and the second primer is selected from the group consisting of SEQ ID NO: 1 and SEQ ID NO: 2, SEQ ID NO: 4 and SEQ ID NO: 2, SEQ ID NO: 4 and SEQ ID NO: 5, SEQ ID NO: 6 and SEQ ID NO: 7, SEQ ID NO: 9 and SEQ ID NO: 10, and SEQ ID NO: 11 and SEQ ID NO: 12;
wherein when the pathogen is infectious pancreatic necrosis virus (IPNV), a sequence combination of the first primer and the second primer is selected from the group consisting of SEQ ID NO: 14 and SEQ ID NO: 15, SEQ ID NO: 17 and SEQ ID NO: 18, SEQ ID NO: 20 and SEQ ID NO: 21, and SEQ ID NO: 23 and SEQ ID NO: 24;
wherein when the pathogen is infectious salmon anemia virus (ISAV), a sequence combination of the first primer and the second primer is selected from the group consisting of SEQ ID NO: 26 and SEQ ID NO: 27, SEQ ID NO: 29 and SEQ ID NO: 30, SEQ ID NO: 32 and SEQ ID NO: 33, SEQ ID NO: 35 and SEQ ID NO: 36, and SEQ ID NO: 37 and SEQ ID NO: 38;
wherein when the pathogen is piscine myocarditis virus (PMCV), a sequence combination of the first primer and the second primer is selected from the group consisting of SEQ ID NO: 39 and SEQ ID NO: 40, SEQ ID NO: 42 and SEQ ID NO: 43, SEQ ID NO: 45 and SEQ ID NO: 46, SEQ ID NO: 48 and SEQ ID NO: 49, SEQ ID NO: 51 and SEQ ID NO: 49, and SEQ ID NO: 52 and SEQ ID NO: 49;
wherein when the pathogen is salmonid alphavirus (SAV), a sequence combination of the first primer and the second primer is selected from the group consisting of SEQ ID NO: 53 and SEQ ID NO: 54, SEQ ID NO: 56 and SEQ ID NO: 57, SEQ ID NO: 59 and SEQ ID NO: 60, SEQ ID NO: 62 and SEQ ID NO: 63, and SEQ ID NO: 65 and SEQ ID NO: 66;
wherein when the pathogen is *Neoparamoeba perurans,* a sequence combination of the first primer and the second primer is selected from the group consisting of SEQ ID NO: 68 and SEQ ID NO: 69, SEQ ID NO: 71 and SEQ ID NO: 72, SEQ ID NO: 74 and SEQ ID NO: 75, and SEQ ID NO: 77 and SEQ ID NO: 78;
wherein when the pathogen is *Candidatus Branchiomonas cysticola* (CBc), a sequence combination of the first primer and the second primer is selected from the group consisting of SEQ ID NO: 80 and SEQ ID NO: 81, and SEQ ID NO: 83 and SEQ ID NO: 84;
wherein when the pathogen is *Desmozoon lepeophtherii* (Des), a sequence combination of the first primer and the second primer is selected from the group consisting of SEQ ID NO: 86 and SEQ ID NO: 87, SEQ ID NO: 89 and SEQ ID NO: 90, SEQ ID NO: 92 and SEQ ID NO: 93, SEQ ID NO: 95 and SEQ ID NO: 97, and SEQ ID NO: 96 and SEQ ID NO: 97;
wherein when the pathogen is salmon gill poxvirus (SGPV), a sequence combination of the first primer and the second primer is selected from the group consisting of SEQ ID NO: 99 and SEQ ID NO: 100, and SEQ ID NO: 102 and SEQ ID NO: 103, and SEQ ID NO: 105 and SEQ ID NO: 106, and SEQ ID NO: 108 and SEQ ID NO: 109;
wherein when the pathogen is *Piscirickettsia salmonis,* a sequence combination of the first primer and the second primer is selected from the group consisting of SEQ ID NO: 111 and SEQ ID NO: 113, and SEQ ID NO: 114 and SEQ ID NO: 115, and SEQ ID NO: 116 and SEQ ID NO: 117, and SEQ ID NO: 119 and SEQ ID NO: 120, and SEQ ID NO: 122 and SEQ ID NO: 123; and
wherein when the pathogen is *Renibacterium salmoninarum,* a sequence combination of the first primer and the second primer is selected from the group consisting of SEQ ID NO: 126 and SEQ ID NO: 128, and SEQ ID NO: 127 and SEQ ID NO: 128, and SEQ ID NO: 130 and SEQ ID NO: 131, and SEQ ID NO: 133 and SEQ ID NO: 134, and SEQ ID NO: 136 and SEQ ID NO: 137.

2. The pair of oligonucleotides of claim 1, further comprising an oligonucleotide probe having an oligonucleotide probe sequence, a fluorescer molecule attached to a first location on the oligonucleotide probe, and a quencher molecule attached to a second location on the oligonucleotide probe.

3. The pair of oligonucleotides of claim 2, wherein the pathogen is PRV, and
when the sequence combination of the first primer and the second primer is SEQ ID NO: 1 and SEQ ID NO: 2, the oligonucleotide probe is a 13- to 25-bp oligonucleotide between the 71st to 95th nucleotides of segment L1 gene of PRV;
when the sequence combination of the first primer and the second primer is SEQ ID NO: 4 and SEQ ID NO: 2, the oligonucleotide probe is a 13- to 25-bp oligonucleotide between the 52nd to 95th nucleotides of segment L1 gene of PRV;
whne the sequence combination of the first primer and the second primer is SEQ ID NO: 4 and SEQ ID NO: 5, the oligonucleotide probe is a 13- to 25-bp oligonucleotide between the 52nd to 100th nucleotides of segment L1 gene of PRV;
when the sequence combination of the first primer and the second primer is SEQ ID NO: 6 and SEQ ID NO: 7, the oligonucleotide probe is a 13- to 35-bp oligonucleotide between the 343rd to 385th nucleotides of the gene of Sigma 3 protein of PRV;
when the sequence combination of the first primer and the second primer is SEQ ID NO: 9 and SEQ ID NO: 10, the oligonucleotide probe is a 13- to 35-bp oligonucleotide between the 350th to 380th nucleotides of the gene of Sigma 3 protein of PRV; and
when the sequence combination of the first primer and the second primer is SEQ ID NO: 11 and SEQ ID NO: 12, the oligonucleotide probe is a 13- to 35-bp oligonucleotide between the 280th to 315th nucleotides of segment L1 gene of PRV.

4. The pair of oligonucleotides of claim 2, wherein the pathogen is PRV, and
when the sequence combination of the first primer and the second primer is selected from the group consisting of SEQ ID NO: 1 and SEQ ID NO: 2, SEQ ID NO: 4 and SEQ ID NO: 2, and SEQ ID NO: 4 and SEQ ID NO: 5, the oligonucleotide probe has a sequence of SEQ ID NO: 3;
when the sequence combination of the first primer and the second primer is selected from the group consisting of SEQ ID NO: 6 and SEQ ID NO: 7, and NO: 9 and SEQ ID NO: 10, the oligonucleotide probe has a sequence of SEQ ID NO: 8;
when the sequence combination of the first primer and the second primer is SEQ ID NO: 11 and SEQ ID NO: 12, the oligonucleotide probe has a sequence of SEQ ID NO: 13.

5. The pair of oligonucleotides of claim 2, wherein the pathogen is IPNV, and
when the sequence combination of the first primer and the second primer is SEQ ID NO: 14 and SEQ ID NO: 15, the oligonucleotide probe is a 13- to 35-bp oligonucleotide between the 2010th to 2057th nucleotides of VPS and polyprotein genes of IPNV;
when the sequence combination of the first primer and the second primer is SEQ ID NO: 17 and SEQ ID NO: 18, the oligonucleotide probe is a 13- to 35-bp oligonucleotide between the 2345th to 2408th nucleotides of VPS and polyprotein genes of IPNV;
when the sequence combination of the first primer and the second primer is SEQ ID NO: 20 and SEQ ID NO: 21, the oligonucleotide probe is a 13- to 35-bp oligonucleotide between the 2533rd to 2580th nucleotides of VPS and polyprotein genes of IPNV; and
when the sequence combination of the first primer and the second primer is SEQ ID NO: 23 and SEQ ID NO: 24, the oligonucleotide probe is a 13- to 35-bp oligonucleotide between the 2775th to 2818th nucleotides of VP5 and polyprotein genes of IPNV.

6. The pair of oligonucleotides of claim 2, wherein the pathogen is IPNV, and
when the sequence combination of the first primer and the second primer is SEQ ID NO: 14 and SEQ ID NO: 15, the oligonucleotide probe has a sequence of SEQ ID NO: 16;
when the sequence combination of the first primer and the second primer is SEQ ID NO: 17 and SEQ ID NO: 18, the oligonucleotide probe has a sequence of SEQ ID NO: 19;
when the sequence combination of the first primer and the second primer is SEQ ID NO: 20 and SEQ ID NO: 21, the oligonucleotide probe has a sequence of SEQ ID NO: 22; and
when the sequence combination of the first primer and the second primer is SEQ ID NO: 23 and SEQ ID NO: 24, the oligonucleotide probe has a sequence of SEQ ID NO: 25.

7. The pair of oligonucleotides of claim 2, wherein the pathogen is ISAV, and
when the pathogen is ISAV, and the sequence combination of the first primer and the second primer is SEQ ID NO: 26 and SEQ ID NO: 27, the oligonucleotide probe is a 13- to 35-bp oligonucleotide between the 282nd to 328th nucleotides of open reading frame (ORF) 1 and ORF2 genes of ISAV;
when the sequence combination of the first primer and the second primer is SEQ ID NO: 29 and SEQ ID NO: 30, the oligonucleotide probe is a 13- to 35-bp oligonucleotide between the 79th to 121st nucleotides of ORF1 and ORF2 genes of ISAV;
when the sequence combination of the first primer and the second primer is SEQ ID NO: 32 and SEQ ID NO: 33, the oligonucleotide probe is a 13- to 30-bp oligonucleotide between the 244th to 273rd nucleotides of ORF1 and ORF2 genes of ISAV;
when the sequence combination of the first primer and the second primer is SEQ ID NO: 35 and SEQ ID NO: 36, the oligonucleotide probe is a 13- to 35-bp oligonucleotide between the 237th to 272nd nucleotides of ORF1 and ORF2 genes of ISAV; and
when the sequence combination of the first primer and the second primer is SEQ ID NO: 37 and SEQ ID NO: 38, the oligonucleotide probe is a 13- to 35-bp oligonucleotide between the 235th to 286th nucleotides of ORF1 and ORF2 genes of ISAV.

8. The pair of oligonucleotides of claim 2, wherein the pathogen is ISAV, and
when the pathogen is ISAV, and the sequence combination of the first primer and the second primer is SEQ ID NO: 26 and SEQ ID NO: 27, the oligonucleotide probe has a sequence of SEQ ID NO: 28;
when the sequence combination of the first primer and the second primer is SEQ ID NO: 29 and SEQ ID NO: 30, the oligonucleotide probe has a sequence of SEQ ID NO: 31; and
when the sequence combination of the first primer and the second primer is selected from the group consisting of SEQ ID NO: 32 and SEQ ID NO: 33, SEQ ID NO: 35 and SEQ ID NO: 36, and SEQ ID NO: 37 and SEQ ID NO: 38, the oligonucleotide probe has a sequence of SEQ ID NO: 34.

9. The pair of oligonucleotides of claim 2, wherein the pathogen is PMCV, and
when the sequence combination of the first primer and the second primer is SEQ ID NO: 39 and SEQ ID NO: 40, the oligonucleotide probe is a 13- to 35-bp oligonucleotide between the 6266th to 6306th nucleotides of PMCV genome;
when the sequence combination of the first primer and the second primer is SEQ ID NO: 42 and SEQ ID NO: 43, the oligonucleotide probe is a 13- to 35-bp oligonucleotide between the 5632nd to 5672nd nucleotides of PMCV genome;
when the sequence combination of the first primer and the second primer is SEQ ID NO: 45 and SEQ ID NO: 46, the oligonucleotide probe is a 13- to 35-bp oligonucleotide between the 5693rd to 5747th nucleotides of PMCV genome;
when the sequence combination of the first primer and the second primer is SEQ ID NO: 48 and SEQ ID NO: 49, the oligonucleotide probe is a 13- to 35-bp oligonucleotide between the 6294th to 6331st nucleotides of PMCV genome;
when the sequence combination of the first primer and the second primer is SEQ ID NO: 51 and SEQ ID NO: 49, the oligonucleotide probe is a 13- to 35-bp oligonucleotide between the 6271st to 6331st nucleotides of PMCV genome; and
when the sequence combination of the first primer and the second primer is SEQ ID NO: 52 and SEQ ID NO: 49, the oligonucleotide probe is a 13- to 35-bp oligonucleotide between the 6290th to 6331st nucleotides of PMCV genome.

10. The pair of oligonucleotides of claim 2, wherein the pathogen is PMCV, and
when the sequence combination of the first primer and the second primer is SEQ ID NO: 39 and SEQ ID NO: 40, the oligonucleotide probe has a sequence of SEQ ID NO: 41;
when the sequence combination of the first primer and the second primer is SEQ ID NO: 42 and SEQ ID NO: 43, the oligonucleotide probe has a sequence of SEQ ID NO: 44;
when the sequence combination of the first primer and the second primer is SEQ ID NO: 45 and SEQ ID NO: 46, the oligonucleotide probe has a sequence of SEQ ID NO: 47; and
when the sequence combination of the first primer and the second primer is selected from the group consisting of SEQ ID NO: 48 and SEQ ID NO: 49, SEQ ID NO: 51 and SEQ ID NO: 49, and SEQ ID NO: 52 and SEQ ID NO: 49, the oligonucleotide probe has a sequence of SEQ ID NO: 50.

11. The pair of oligonucleotides of claim 2, wherein the pathogen is SAV, and
when the sequence combination of the first primer and the second primer is SEQ ID NO: 53 and SEQ ID NO: 54, the oligonucleotide probe is a 13- to 35-bp oligonucleotide between the 611st to 653rd nucleotides of SAV genome;
when the sequence combination of the first primer and the second primer is SEQ ID NO: 56 and SEQ ID NO: 57, the oligonucleotide probe is a 13- to 35-bp oligonucleotide between the 145th to 181st nucleotides of SAV genome;
when the sequence combination of the first primer and the second primer is SEQ ID NO: 59 and SEQ ID NO: 60, the oligonucleotide probe is a 13- to 35-bp oligonucleotide between the 2691st to 2761st nucleotides of SAV genome;
when the sequence combination of the first primer and the second primer is SEQ ID NO: 62 and SEQ ID NO: 63, the oligonucleotide probe is a 13- to 35-bp oligonucleotide between the 1032nd to 1075th nucleotides of SAV genome; and
when the sequence combination of the first primer and the second primer is SEQ ID NO: 65 and SEQ ID NO: 66, the oligonucleotide probe is a 13- to 35-bp oligonucleotide between the 969th to 1011st nucleotides of SAV genome.

12. The pair of oligonucleotides of claim 2, wherein the pathogen is SAV, and
when the sequence combination of the first primer and the second primer is SEQ ID NO: 53 and SEQ ID NO: 54, the oligonucleotide probe has a sequence of SEQ ID NO: 55;
when the sequence combination of the first primer and the second primer is SEQ ID NO: 56 and SEQ ID NO: 57, the oligonucleotide probe has a sequence of SEQ ID NO: 58;
when the sequence combination of the first primer and the second primer is SEQ ID NO: 59 and SEQ ID NO: 60, the oligonucleotide probe has a sequence of SEQ ID NO: 61;
when the sequence combination of the first primer and the second primer is SEQ ID NO: 62 and SEQ ID NO: 63, the oligonucleotide probe has a sequence of SEQ ID NO: 64; and
when the sequence combination of the first primer and the second primer is SEQ ID NO: 65 and SEQ ID NO: 66, the oligonucleotide probe has a sequence of SEQ ID NO: 67.

13. The pair of oligonucleotides of claim 2, wherein the pathogen is *N. perurans,* and
when the sequence combination of the first primer and the second primer is SEQ ID NO: 68 and SEQ ID NO: 69, the oligonucleotide probe is a 13- to 35-bp oligonucleotide between the 462nd to 520th nucleotides of 18S ribosomal RNA (rRNA) of *N. perurans;*
when the sequence combination of the first primer and the second primer is SEQ ID NO: 71 and SEQ ID NO: 72, the oligonucleotide probe is a 13- to 35-bp oligonucleotide between the 355th to 405th nucleotides of 18S rRNA of *N. perurans;*
when the sequence combination of the first primer and the second primer is SEQ ID NO: 74 and SEQ ID NO: 75, the oligonucleotide probe is a 13- to 35-bp oligonucleotide between the 234th to 281st nucleotides of 18S rRNA of *N. perurans;* and
when the sequence combination of the first primer and the second primer is SEQ ID NO: 77 and SEQ ID NO: 78, the oligonucleotide probe is a 13- to 24-bp oligonucleotide between the 357th to 380th nucleotides of 18S rRNA of *N. perurans.*

14. The pair of oligonucleotides of claim 2, wherein the pathogen is *N. perurans,* and
when the sequence combination of the first primer and the second primer is SEQ ID NO: 68 and SEQ ID NO: 69, the oligonucleotide probe has a sequence of SEQ ID NO: 70;
when the sequence combination of the first primer and the second primer is SEQ ID NO: 71 and SEQ ID NO: 72, the oligonucleotide probe has a sequence of SEQ ID NO: 73;
when the sequence combination of the first primer and the second primer is SEQ ID NO: 74 and SEQ ID NO: 75, the oligonucleotide probe has a sequence of SEQ ID NO: 76; and
when the sequence combination of the first primer and the second primer is SEQ ID NO: 77 and SEQ ID NO: 78, the oligonucleotide probe has a sequence of SEQ ID NO: 79.

15. The pair of oligonucleotides of claim 2, wherein the pathogen is *C. B. cysticola* and
when the sequence combination of the first primer and the second primer is SEQ ID NO: 80 and SEQ ID NO: 81, the oligonucleotide probe is a 13- to 35-bp oligonucleotide between the 1068th to 1125th nucleotides of 16S ribosomal RNA (rRNA) of *C*. *B. cysticola;* and
when the sequence combination of the first primer and the second primer is SEQ ID NO: 83 and SEQ ID NO: 84, the oligonucleotide probe is a 13- to 30-bp oligonucleotide between the 1212nd to 1241th nucleotides of 16S rRNA of *C*. *B. cysticola.*

16. The pair of oligonucleotides of claim 2, wherein the pathogen is *C*. *B. cysticola,* and
when the sequence combination of the first primer and the second primer is SEQ ID NO: 80 and SEQ ID NO: 81, the oligonucleotide probe has a sequence of SEQ ID NO: 82; and
when the sequence combination of the first primer and the second primer is SEQ ID NO: 83 and SEQ ID NO: 84, the oligonucleotide probe has a sequence of SEQ ID NO: 85.

17. The pair of oligonucleotides of claim 2, wherein the pathogen is *D. lepeophtherii,* and
when the sequence combination of the first primer and the second primer is SEQ ID NO: 86 and SEQ ID NO: 87, the oligonucleotide probe is a 13- to 35-bp oligonucleotide between the 967th to 1010th nucleotides of 16S ribosomal RNA (rRNA) of *D. lepeophtherii;*
when the sequence combination of the first primer and the second primer is SEQ ID NO: 89 and SEQ ID NO: 90, the oligonucleotide probe is a 13- to 35-bp oligonucleotide between the 1246th to 1285th nucleotides of 16S rRNA of *D. lepeophtherii;*
when the sequence combination of the first primer and the second primer is SEQ ID NO: 92 and SEQ ID NO: 93, the oligonucleotide probe is a 13- to 35-bp oligonucleotide between the 1366th to 1414th nucleotides of 16S rRNA of *D. lepeophtherii;*
when the sequence combination of the first primer and the second primer is SEQ ID NO: 95 and SEQ ID NO: 97, the oligonucleotide probe is a 13- to 35-bp oligonucleotide between the 815th to 859th nucleotides of 16S rRNA of *D. lepeophtherii;* and
when the sequence combination of the first primer and the second primer is SEQ ID NO: 96 and SEQ ID NO: 97, the oligonucleotide probe is a 13- to 35-bp oligonucleotide between the 817th to 859th nucleotides of 16S rRNA of *D. lepeophtherii.*

18. The pair of oligonucleotides of claim 2, wherein the pathogen is *D. lepeophtherii,* and
when the sequence combination of the first primer and the second primer is SEQ ID NO: 86 and SEQ ID NO: 87, the oligonucleotide probe has a sequence of SEQ ID NO: 88;
when the sequence combination of the first primer and the second primer is SEQ ID NO: 89 and SEQ ID NO: 90, the oligonucleotide probe has a sequence of SEQ ID NO: 91;
when the sequence combination of the first primer and the second primer is SEQ ID NO: 92 and SEQ ID NO: 93, the oligonucleotide probe has a sequence of SEQ ID NO: 94; and
when the sequence combination of the first primer and the second primer is selected from the group consisting of SEQ ID NO: 95 and SEQ ID NO: 97, and SEQ ID NO: 96 and SEQ ID NO: 97, the oligonucleotide probe has a sequence of SEQ ID NO: 98.

19. The pair of oligonucleotides of claim 2, wherein the pathogen is SGPV, and
when the sequence combination of the first primer and the second primer is SEQ ID NO: 99 and SEQ ID NO: 100, the oligonucleotide probe is a 13- to 35-bp oligonucleotide between the 99272nd to 99318th nucleotides of SGPV genome;
when the sequence combination of the first primer and the second primer is SEQ ID NO: 102 and SEQ ID NO: 103, the oligonucleotide probe is a 13- to 27-bp oligonucleotide between the 123213rd to 123239th nucleotides of SGPV genome;
when the sequence combination of the first primer and the second primer is SEQ ID NO: 105 and SEQ ID NO: 106, the oligonucleotide probe is a 13- to 30-bp oligonucleotide between the 123202nd to 123232nd nucleotides of SGPV genome; and
when the sequence combination of the first primer and the second primer is SEQ ID NO: 108 and SEQ ID NO: 109, the oligonucleotide probe is a 13- to 35-bp oligonucleotide between the 123556th to 123610th nucleotides of SGPV genome.

20. The pair of oligonucleotides of claim 2, wherein the pathogen is SGPV, and
when the sequence combination of the first primer and the second primer is SEQ ID NO: 99 and SEQ ID NO: 100, the oligonucleotide probe has a sequence of SEQ ID NO: 101;
when the sequence combination of the first primer and the second primer is SEQ ID NO: 102 and SEQ ID NO: 103, the oligonucleotide probe has a sequence of SEQ ID NO: 104;
when the sequence combination of the first primer and the second primer is SEQ ID NO: 105 and SEQ ID NO: 106, the oligonucleotide probe has a sequence of SEQ ID NO: 107; and
when the sequence combination of the first primer and the second primer is SEQ ID NO: 108 and SEQ ID NO: 109, the oligonucleotide probe has a sequence of SEQ ID NO: 110.

21. The pair of oligonucleotides of claim 2, wherein the pathogen is *P. salmonis,* and
when the sequence combination of the first primer and the second primer is SEQ ID NO: 111 and SEQ ID NO: 112, the oligonucleotide probe is a 13- to 35-bp oligonucleotide between the 952nd to 1065th nucleotides of 16S ribosomal RNA (rRNA) of *P. salmonis;*
when the sequence combination of the first primer and the second primer is SEQ ID NO: 114 and SEQ ID NO: 115, the oligonucleotide probe is a 13- to 35-bp oligonucleotide between the 1014th to 1050th nucleotides of 16S rRNA of *P. salmonis;*
when the sequence combination of the first primer and the second primer is SEQ ID NO: 116 and SEQ ID NO: 117, the oligonucleotide probe is a 13- to 17-bp oligonucleotide between the 315th to 331st nucleotides of 16S rRNA of *P. salmonis;*
when the sequence combination of the first primer and the second primer is SEQ ID NO: 119 and SEQ ID NO: 120, the oligonucleotide probe is a 13- to 17-bp oligonucleotide between the 529th to 545th nucleotides of 16S rRNA of *P. salmonis;* and
when the sequence combination of the first primer and the second primer is SEQ ID NO: 122 and SEQ ID NO: 123, the oligonucleotide probe is a 13- to 20-bp oligonucleotide between the 1181st to 1200th nucleotides of 16S rRNA of *P. salmonis.*

22. The pair of oligonucleotides of claim 2, wherein the pathogen is *P. salmonis,* and
when the sequence combination of the first primer and the second primer is selected from the group consisting of SEQ ID NO: 111 and SEQ ID NO: 112, and SEQ ID NO: 114 and SEQ ID NO: 115, the oligonucleotide probe has a sequence of SEQ ID NO: 113;
when the sequence combination of the first primer and the second primer is SEQ ID NO: 116 and SEQ ID NO: 117, the oligonucleotide probe has a sequence of SEQ ID NO: 118;
when the sequence combination of the first primer and the second primer is SEQ ID NO: 119 and SEQ ID NO: 120, the oligonucleotide probe has a sequence of SEQ ID NO: 121; and
when the sequence combination of the first primer and the second primer is SEQ ID NO: 122 and SEQ ID NO: 123, the oligonucleotide probe has a sequence of SEQ ID NO: 124.

23. The pair of oligonucleotides of claim 2, wherein the pathogen is *R. salmoninarum,* and
when the sequence combination of the first primer and the second primer is SEQ ID NO: 125 and SEQ ID NO: 128, the oligonucleotide probe is a 13- to 31-bp oligonucleotide between the 1035th to 1065th nucleotides of 57KD extracellular protein precursor (ECP) of *R. salmoninarum;*
when the sequence combination of the first primer and the second primer is SEQ ID NO: 126 and SEQ ID NO: 128, the oligonucleotide probe is a 13- to 35-bp oligonucleotide between the 1024th to 1065th nucleotides of 57KD ECP of *R. salmoninarum;*
when the sequence combination of the first primer and the second primer is SEQ ID NO: 127 and SEQ ID NO: 128, the oligonucleotide probe is a 13- to 29-bp oligonucleotide between the 1037th to 1065th nucleotides of 57KD ECP of *R. salmoninarum;*
when the sequence combination of the first primer and the second primer is SEQ ID NO: 130 and SEQ ID NO: 131, the oligonucleotide probe is a 13- to 25-bp oligonucleotide between the 782nd to 806th nucleotides of 57KD ECP of *R. salmoninarum*;
when the sequence combination of the first primer and the second primer is SEQ ID NO: 133 and SEQ ID NO: 134, the oligonucleotide probe is a 13- to 35-bp oligonucleotide between the 880th to 964th nucleotides of 57KD ECP of *R. salmoninarum;* and
when the sequence combination of the first primer and the second primer is SEQ ID NO: 136 and SEQ ID NO: 137, the oligonucleotide probe is a 13- to 35-bp oligonucleotide between the 646th to 738th nucleotides of 57KD ECP of *R. salmoninarum.*

24. The pair of oligonucleotides of claim 2, wherein the pathogen is *R. salmoninarum,* and
when the sequence combination of the first primer and the second primer is selected from the group consisting of SEQ ID NO: 125 and SEQ ID NO: 128, SEQ ID NO: 126 and SEQ ID NO: 128, and SEQ ID NO: 127 and SEQ ID NO: 128, the oligonucleotide probe has a sequence of SEQ ID NO: 129;
when the sequence combination of the first primer and the second primer is SEQ ID NO: 130 and SEQ ID NO: 131, the oligonucleotide probe has a sequence of SEQ ID NO: 132;
when the sequence combination of the first primer and the second primer is SEQ ID NO: 133 and SEQ ID NO: 134, the oligonucleotide probe has a sequence of SEQ ID NO: 135; and
when the sequence combination of the first primer and the second primer is SEQ ID NO: 136 and SEQ ID NO: 137, the oligonucleotide probe has a sequence of SEQ ID NO: 138.


SEQUENCE LISTING

<110>  Schweitzer Biotech Company Ltd.

<120>  Methods for Detecting Pathogens in Fish

<130>  PCT/CN2019/108518

<150>  US 62/741,635
<151>  2018-10-05

<160>  138

<170>  PatentIn version 3.5

<210>  1
<211>  22
<212>  DNA
<213>  Artificial Sequence

<220>
<223>  primer

<400>  1
acttgaaaac ctgagtgtgg ca                                                22


<210>  2
<211>  23
<212>  DNA
<213>  Artificial Sequence

<220>
<223>  primer

<400>  2
actcgcgtta gtccagatat cag                                               23


<210>  3
<211>  19
<212>  DNA
<213>  Artificial Sequence

<220>
<223>  probe

<400>  3
aagtggcact atcgaatta                                                    19


<210>  4
<211>  25
<212>  DNA
<213>  Artificial Sequence

<220>

<223>  primer

<400>  4
ttgtcaacca actacctgaa gactt                                            25


<210>  5
<211>  24
<212>  DNA
<213>  Artificial Sequence

<220>
<223>  primer

<400>  5
agttttactc gcgttagtcc agat                                             24


<210>  6
<211>  20
<212>  DNA
<213>  Artificial Sequence

<220>
<223>  primer

<400>  6
acgcagacgc ttacctcaga                                                  20


<210>  7
<211>  20
<212>  DNA
<213>  Artificial Sequence

<220>
<223>  primer

<400>  7
gcgtctgtct ggtctgtcgt                                                  20


<210>  8
<211>  23
<212>  DNA
<213>  Artificial Sequence

<220>
<223>  probe

<400>  8
tggacagaag acaacagtcg cgg                                              23


<210>  9
<211>  23
<212>  DNA

<213>  Artificial Sequence

<220>
<223>  primer

<400>  9
agacgcttac ctcagaatcc tca                                              23


<210>  10
<211>  23
<212>  DNA
<213>  Artificial Sequence

<220>
<223>  primer

<400>  10
gtctgtctgg tctgtcgttt gaa                                              23


<210>  11
<211>  20
<212>  DNA
<213>  Artificial Sequence

<220>
<223>  primer

<400>  11
gttgtgcgtg tgcctaagtg                                                  20


<210>  12
<211>  20
<212>  DNA
<213>  Artificial Sequence

<220>
<223>  primer

<400>  12
tttccagcac gtcagtgaga                                                  20


<210>  13
<211>  19
<212>  DNA
<213>  Artificial Sequence

<220>
<223>  probe

<400>  13
tgatgatgtc tacctccct                                                   19

<210>  14
<211>  18
<212>  DNA
<213>  Artificial Sequence

<220>
<223>  primer

<400>  14
ttgctgggcc aattatgg                                                    18


<210>  15
<211>  20
<212>  DNA
<213>  Artificial Sequence

<220>
<223>  primer

<400>  15
caccatcctt ggaactccgt                                                  20


<210>  16
<211>  25
<212>  DNA
<213>  Artificial Sequence

<220>
<223>  probe

<400>  16
ccctctgctc aactaggact gtccc                                            25


<210>  17
<211>  20
<212>  DNA
<213>  Artificial Sequence

<220>
<223>  primer

<400>  17
ggatggacga ggaactgcaa                                                  20


<210>  18
<211>  20
<212>  DNA
<213>  Artificial Sequence

<220>
<223>  primer

<400>  18

ggtccatgcc atgagcctaa 20

<210> 19
<211> 19
<212> DNA
<213> Artificial Sequence

<220>
<223> probe

<400> 19
acgccacaat ggcacgggc 19

<210> 20
<211> 20
<212> DNA
<213> Artificial Sequence

<220>
<223> primer

<400> 20
ccaccaaagc accctgagaa 20

<210> 21
<211> 20
<212> DNA
<213> Artificial Sequence

<220>
<223> primer

<400> 21
tcacggcgtc cagtgatatg 20

<210> 22
<211> 21
<212> DNA
<213> Artificial Sequence

<220>
<223> probe

<400> 22
acgcccaaga ggcgagagcc a 21

<210> 23
<211> 21
<212> DNA
<213> Artificial Sequence

<220>

<223>  primer

<400>  23
cgaccgacat gaacaaaatc a                                                21


<210>  24
<211>  26
<212>  DNA
<213>  Artificial Sequence

<220>
<223>  primer

<400>  24
actgcatcgt agaactcctc tggtgc                                           26


<210>  25
<211>  26
<212>  DNA
<213>  Artificial Sequence

<220>
<223>  probe

<400>  25
ctagccaaca gtgtgtacgg cctccc                                           26


<210>  26
<211>  20
<212>  DNA
<213>  Artificial Sequence

<220>
<223>  primer

<400>  26
gcgacgatga ccctctactg                                                  20


<210>  27
<211>  20
<212>  DNA
<213>  Artificial Sequence

<220>
<223>  primer

<400>  27
cagccagttc ctgatgtggt                                                  20


<210>  28
<211>  16
<212>  DNA

<213>  Artificial Sequence

<220>
<223>  probe

<400>  28
ttgccatgct tcccga                                                        16


<210>  29
<211>  20
<212>  DNA
<213>  Artificial Sequence

<220>
<223>  primer

<400>  29
gcatgagaga agcaaaccca                                                    20


<210>  30
<211>  20
<212>  DNA
<213>  Artificial Sequence

<220>
<223>  primer

<400>  30
tgcgtaaacc acctctggtc                                                    20


<210>  31
<211>  25
<212>  DNA
<213>  Artificial Sequence

<220>
<223>  probe

<400>  31
accacgggag ctgatcagac atgcc                                              25


<210>  32
<211>  22
<212>  DNA
<213>  Artificial Sequence

<220>
<223>  primer

<400>  32
caaaaccttg aaccaattgg tg                                                 22

<210>  33
<211>  25
<212>  DNA
<213>  Artificial Sequence

<220>
<223>  primer

<400>  33
gtggatcttt catcacacag tagag                                              25


<210>  34
<211>  22
<212>  DNA
<213>  Artificial Sequence

<220>
<223>  probe

<400>  34
cggaatgatc agtcgagcga cg                                                 22


<210>  35
<211>  22
<212>  DNA
<213>  Artificial Sequence

<220>
<223>  primer

<400>  35
tgaaagtcaa aaccttgaac ca                                                 22


<210>  36
<211>  25
<212>  DNA
<213>  Artificial Sequence

<220>
<223>  primer

<400>  36
tggatctttc atcacacagt agaga                                              25


<210>  37
<211>  24
<212>  DNA
<213>  Artificial Sequence

<220>
<223>  primer

<400>  37

atggtgaaag tcaaaacctt gaac 24

<210> 38
<211> 20
<212> DNA
<213> Artificial Scqucncc

<220>
<223> primer

<400> 38
tccagacggt ggatctttca 20

<210> 39
<211> 20
<212> DNA
<213> Artificial Sequence

<220>
<223> primcr

<400> 39
gaggaccaac ccagaaccag 20

<210> 40
<211> 20
<212> DNA
<213> Artificial Sequence

<220>
<223> primer

<400> 40
tatggagggc gtggctctaa 20

<210> 41
<211> 16
<212> DNA
<213> Artificial Sequence

<220>
<223> probe

<400> 41
ccctcatctc gtgttc 16

<210> 42
<211> 20
<212> DNA
<213> Artificial Sequence

<220>

<223>  primer

<400>  42
tgttcaggcg agaatggtgt                                                    20


<210>  43
<211>  20
<212>  DNA
<213>  Artificial Sequence

<220>
<223>  primer

<400>  43
cgatcttctc agcccttccc                                                    20


<210>  44
<211>  21
<212>  DNA
<213>  Artificial Sequence

<220>
<223>  probe

<400>  44
tcgcattccc tgcagtgcac a                                                  21


<210>  45
<211>  20
<212>  DNA
<213>  Artificial Sequence

<220>
<223>  primer

<400>  45
gggaagggct gagaagatcg                                                    20


<210>  46
<211>  20
<212>  DNA
<213>  Artificial Sequence

<220>
<223>  primer

<400>  46
cgtagcacca cctctttccc                                                    20


<210>  47
<211>  23
<212>  DNA

<213>  Artificial Sequence

<220>
<223>  probe

<400>  47
tcttcccaca tgtggcgctc tga                                              23


<210>  48
<211>  20
<212>  DNA
<213>  Artificial Sequence

<220>
<223>  primer

<400>  48
gaggaacacg agatgaggga                                                  20


<210>  49
<211>  22
<212>  DNA
<213>  Artificial Sequence

<220>
<223>  primer

<400>  49
tcaaactcaa cacccatctg tc                                               22


<210>  50
<211>  14
<212>  DNA
<213>  Artificial Sequence

<220>
<223>  probe

<400>  50
agccacgccc tcca                                                        14


<210>  51
<211>  17
<212>  DNA
<213>  Artificial Sequence

<220>
<223>  primer

<400>  51
acccagaacc agcggaa                                                     17

<210> 52
<211> 19
<212> DNA
<213> Artificial Sequence

<220>
<223> primer

<400> 52
ccggaggaac acgagatga 19


<210> 53
<211> 22
<212> DNA
<213> Artificial Sequence

<220>
<223> primer

<400> 53
gtggtatact ggatcgggtt cg 22


<210> 54
<211> 22
<212> DNA
<213> Artificial Sequence

<220>
<223> primer

<400> 54
gctccagttg gcgtcatata gc 22


<210> 55
<211> 22
<212> DNA
<213> Artificial Sequence

<220>
<223> probe

<400> 55
ccgttcgtgt tccacacgga cg 22


<210> 56
<211> 20
<212> DNA
<213> Artificial Sequence

<220>
<223> primer

<400> 56

ttccagggtt cgaagtggtg                                                    20

<210>  57
<211>  20
<212>  DNA
<213>  Artificial Sequence

<220>
<223>  primer

<400>  57
agccaagtgg gagaaagctc                                                    20

<210>  58
<211>  18
<212>  DNA
<213>  Artificial Sequence

<220>
<223>  probe

<400>  58
aacgaccatg ccgccgcc                                                      18

<210>  59
<211>  19
<212>  DNA
<213>  Artificial Sequence

<220>
<223>  primer

<400>  59
cggccatcgt ttcaaacct                                                     19

<210>  60
<211>  20
<212>  DNA
<213>  Artificial Sequence

<220>
<223>  primer

<400>  60
cgcttctttg tcaggcttgg                                                    20

<210>  61
<211>  22
<212>  DNA
<213>  Artificial Sequence

<220>

<223>  probe

<400>  61
cgaacccgtg caagaagccg gt                                                22


<210>  62
<211>  20
<212>  DNA
<213>  Artificial Sequence

<220>
<223>  primer

<400>  62
ggagtggtgg tgaccaagtt                                                   20


<210>  63
<211>  20
<212>  DNA
<213>  Artificial Sequence

<220>
<223>  primer

<400>  63
gatgacggca gggatgtagg                                                   20


<210>  64
<211>  26
<212>  DNA
<213>  Artificial Sequence

<220>
<223>  probe

<400>  64
ccacgaacag gtgtcattcc cagtgg                                            26


<210>  65
<211>  20
<212>  DNA
<213>  Artificial Sequence

<220>
<223>  primer

<400>  65
atctgtgagg gcatcgttgg                                                   20


<210>  66
<211>  20
<212>  DNA

<213>  Artificial Sequence

<220>
<223>  primer

<400>  66
aacttggtca ccaccactcc                                                    20


<210>  67
<211>  18
<212>  DNA
<213>  Artificial Sequence

<220>
<223>  probe

<400>  67
cacttcgtac caccgtga                                                      18


<210>  68
<211>  24
<212>  DNA
<213>  Artificial Sequence

<220>
<223>  primer

<400>  68
gttgttgcag ttaaaaagct cgta                                               24


<210>  69
<211>  28
<212>  DNA
<213>  Artificial Sequence

<220>
<223>  primer

<400>  69
gtgaaagtct agtaaggaga agtacata                                           28


<210>  70
<211>  21
<212>  DNA
<213>  Artificial Sequence

<220>
<223>  probe

<400>  70
tggattttga agtttacata t                                                  21

<210>  71
<211>  26
<212>  DNA
<213>  Artificial Sequence

<220>
<223>  primer

<400>  71
gtctggtaat tggaatgagt acaatc                                          26


<210>  72
<211>  18
<212>  DNA
<213>  Artificial Sequence

<220>
<223>  primer

<400>  72
ttggagctgg aattaccg                                                   18


<210>  73
<211>  27
<212>  DNA
<213>  Artificial Sequence

<220>
<223>  probe

<400>  73
cgagtatcaa ttggagggca agtctgg                                         27


<210>  74
<211>  21
<212>  DNA
<213>  Artificial Sequence

<220>
<223>  primer

<400>  74
cggagaagga gcctgagaaa c                                               21


<210>  75
<211>  20
<212>  DNA
<213>  Artificial Sequence

<220>
<223>  primer

<400>  75

tgtcactacc tccccgtgtc                                                    20

<210>  76
<211>  16
<212>  DNA
<213>  Artificial Sequence

<220>
<223>  probe

<400>  76
cgcgcaaatt acccaa                                                        16

<210>  77
<211>  28
<212>  DNA
<213>  Artificial Sequence

<220>
<223>  primer

<400>  77
gtctggtaat tggaatgagt acaatcta                                           28

<210>  78
<211>  18
<212>  DNA
<213>  Artificial Sequence

<220>
<223>  primer

<400>  78
tggcaccaga cttgccct                                                      18

<210>  79
<211>  19
<212>  DNA
<213>  Artificial Sequcncc

<220>
<223>  probe

<400>  79
cctttaacga gtatcaatt                                                     19

<210>  80
<211>  20
<212>  DNA
<213>  Artificial Sequence

<220>

<223>  primer

<400>  80
ctcgtgtcgt gagatgttgg                                                    20


<210>  81
<211>  20
<212>  DNA
<213>  Artificial Sequence

<220>
<223>  primer

<400>  81
atttgtcacc ggcagtatcg                                                    20


<210>  82
<211>  20
<212>  DNA
<213>  Artificial Sequence

<220>
<223>  probe

<400>  82
cccgcaacga gcggaaccct                                                    20


<210>  83
<211>  20
<212>  DNA
<213>  Artificial Sequence

<220>
<223>  primer

<400>  83
agggcttcac acgtcataca                                                    20


<210>  84
<211>  20
<212>  DNA
<213>  Artificial Sequence

<220>
<223>  primer

<400>  84
ttctgagatt cgctccacct                                                    20


<210>  85
<211>  21
<212>  DNA

<213>  Artificial Sequence

<220>
<223>  probe

<400>  85
cgcggtttcg ctgccctctg t                                                21


<210>  86
<211>  23
<212>  DNA
<213>  Artificial Sequence

<220>
<223>  primer

<400>  86
gaaggctata acaggtccgt gat                                              23


<210>  87
<211>  25
<212>  DNA
<213>  Artificial Sequence

<220>
<223>  primer

<400>  87
atccgtaagc atatttatgt cgctc                                            25


<210>  88
<211>  19
<212>  DNA
<213>  Artificial Sequence

<220>
<223>  probe

<400>  88
cagatgccct gggccgcaa                                                   19


<210>  89
<211>  20
<212>  DNA
<213>  Artificial Sequence

<220>
<223>  primer

<400>  89
gattggagaa ccagccgttg                                                  20

<210>  90
<211>  22
<212>  DNA
<213>  Artificial Sequence

<220>
<223>  primer

<400>  90
gtgcaaatct gaacgtgatg ct                                                22


<210>  91
<211>  21
<212>  DNA
<213>  Artificial Sequence

<220>
<223>  probe

<400>  91
tcaggatgca ctgccgccaa a                                                 21


<210>  92
<211>  22
<212>  DNA
<213>  Artificial Sequence

<220>
<223>  primer

<400>  92
ggactgctta gtgagtgtgt gt                                                22


<210>  93
<211>  20
<212>  DNA
<213>  Artificial Sequence

<220>
<223>  primer

<400>  93
acacgtttcg cactcgtatg                                                   20


<210>  94
<211>  25
<212>  DNA
<213>  Artificial Sequence

<220>
<223>  probe

<400>  94

cgttcgtcat cgctaccaga gtcga 25

<210> 95
<211> 22
<212> DNA
<213> Artificial Sequence

<220>
<223> primer

<400> 95
aaaacttacc agggccaagt ct 22

<210> 96
<211> 22
<212> DNA
<213> Artificial Sequence

<220>
<223> primer

<400> 96
aacttaccag ggccaagtct ct 22

<210> 97
<211> 22
<212> DNA
<213> Artificial Sequence

<220>
<223> primer

<400> 97
aggtcatccc atcaatttcc aa 22

<210> 98
<211> 26
<212> DNA
<213> Artificial Scqucncc

<220>
<223> probe

<400> 98
cgtagattgg cgacatgaga ggggca 26

<210> 99
<211> 18
<212> DNA
<213> Artificial Sequence

<220>

<223>  primer

<400>  99
cgggtgctcg gattgttt                                                    18


<210>  100
<211>  19
<212>  DNA
<213>  Artificial Sequence

<220>
<223>  primer

<400>  100
gggacctcgt acatgccat                                                   19


<210>  101
<211>  25
<212>  DNA
<213>  Artificial Sequence

<220>
<223>  probe

<400>  101
cgtggccttc atgtattcga ctccg                                            25


<210>  102
<211>  22
<212>  DNA
<213>  Artificial Sequence

<220>
<223>  primer

<400>  102
acggtatcag tcttgtctgc tg                                               22


<210>  103
<211>  25
<212>  DNA
<213>  Artificial Sequence

<220>
<223>  primer

<400>  103
tccaaattca ttatctcctc gtgtc                                            25


<210>  104
<211>  25
<212>  DNA

<213>  Artificial Sequence

<220>
<223>  probe

<400>  104
actctccgac cggcttgatg tttct                                          25


<210>  105
<211>  26
<212>  DNA
<213>  Artificial Sequence

<220>
<223>  primer

<400>  105
taattcctaa aatggacggt atcagt                                         26


<210>  106
<211>  25
<212>  DNA
<213>  Artificial Sequence

<220>
<223>  primer

<400>  106
tcattatctc ctcgtgtcag aaaca                                          25


<210>  107
<211>  23
<212>  DNA
<213>  Artificial Sequence

<220>
<223>  probe

<400>  107
tgctggaact ctccgaccgg ctt                                            23


<210>  108
<211>  26
<212>  DNA
<213>  Artificial Sequence

<220>
<223>  primer

<400>  108
actctgataa catttctgtg tccact                                         26

<210>  109
<211>  25
<212>  DNA
<213>  Artificial Sequence

<220>
<223>  primer

<400>  109
tctaatttac ggtcttcgca tttca                                          25


<210>  110
<211>  25
<212>  DNA
<213>  Artificial Sequence

<220>
<223>  probe

<400>  110
tccattgtcc ctgtatgcca gtcgt                                          25


<210>  111
<211>  18
<212>  DNA
<213>  Artificial Sequence

<220>
<223>  primer

<400>  111
agggagactg ccggtgat                                                  18


<210>  112
<211>  19
<212>  DNA
<213>  Artificial Sequence

<220>
<223>  primer

<400>  112
actacgaggc gctttctca                                                 19


<210>  113
<211>  19
<212>  DNA
<213>  Artificial Sequence

<220>
<223>  probe

<400>  113

ggggcgtaca gacggaggc                                                    19

<210>  114
<211>  22
<212>  DNA
<213>  Artificial Sequence

<220>
<223>  primer

<400>  114
ccttacgacc agggctacac ac                                                 22

<210>  115
<211>  21
<212>  DNA
<213>  Artificial Sequence

<220>
<223>  primer

<400>  115
ttctcaggtt cgctccacat c                                                  21

<210>  116
<211>  16
<212>  DNA
<213>  Artificial Sequence

<220>
<223>  primer

<400>  116
ccagcagccg cggtaa                                                        16

<210>  117
<211>  21
<212>  DNA
<213>  Artificial Scqucncc

<220>
<223>  primer

<400>  117
acgcccagta attccgatta a                                                  21

<210>  118
<211>  14
<212>  DNA
<213>  Artificial Sequence

<220>

<223>  probe

<400>  118
cggagggtgc gagc                                                          14


<210>  119
<211>  20
<212>  DNA
<213>  Artificial Sequence

<220>
<223>  primer

<400>  119
aaggcgactt tctggatcaa                                                    20


<210>  120
<211>  19
<212>  DNA
<213>  Artificial Sequence

<220>
<223>  primer

<400>  120
ggtcgctacc catgctttc                                                     19


<210>  121
<211>  15
<212>  DNA
<213>  Artificial Sequence

<220>
<223>  probe

<400>  121
actgacgctg aggcg                                                         15


<210>  122
<211>  18
<212>  DNA
<213>  Artificial Sequence

<220>
<223>  primer

<400>  122
ttcccgggcc ttgtacac                                                      18


<210>  123
<211>  20
<212>  DNA

<213>  Artificial Sequence

<220>
<223>  primer

<400>  123
cccttctggt gcaattcact                                                    20


<210>  124
<211>  13
<212>  DNA
<213>  Artificial Sequence

<220>
<223>  probe

<400>  124
ccgcccgtca cac                                                           13


<210>  125
<211>  22
<212>  DNA
<213>  Artificial Sequence

<220>
<223>  primer

<400>  125
gtgaccaaca cccagatatc ca                                                 22


<210>  126
<211>  24
<212>  DNA
<213>  Artificial Sequence

<220>
<223>  primer

<400>  126
gaatacgtgg gtagtgacca acac                                               24


<210>  127
<211>  22
<212>  DNA
<213>  Artificial Sequence

<220>
<223>  primer

<400>  127
gaccaacacc cagatatcca tg                                                 22

<210>  128
<211>  19
<212>  DNA
<213>  Artificial Sequence

<220>
<223>  primer

<400>  128
tgtcgccaga ccaccattt                                                    19


<210>  129
<211>  16
<212>  DNA
<213>  Artificial Sequence

<220>
<223>  probe

<400>  129
caccagatgg agcaac                                                       16


<210>  130
<211>  20
<212>  DNA
<213>  Artificial Sequence

<220>
<223>  primer

<400>  130
agcagcaact ctcgagccta                                                   20


<210>  131
<211>  20
<212>  DNA
<213>  Artificial Sequence

<220>
<223>  primer

<400>  131
cgccattcac cttcaccaca                                                   20


<210>  132
<211>  23
<212>  DNA
<213>  Artificial Sequence

<220>
<223>  probe

<400>  132

accgccgtca cgaggcccag tct                                                23

<210>  133
<211>  20
<212>  DNA
<213>  Artificial Sequence

<220>
<223>  primer

<400>  133
gagacaacga ggcaacggaa                                                    20

<210>  134
<211>  20
<212>  DNA
<213>  Artificial Sequence

<220>
<223>  primer

<400>  134
tgttgcggaa ccgttaggtg                                                    20

<210>  135
<211>  24
<212>  DNA
<213>  Artificial Sequence

<220>
<223>  probe

<400>  135
accgggccag cggcatctac tgct                                               24

<210>  136
<211>  20
<212>  DNA
<213>  Artificial Sequence

<220>
<223>  primer

<400>  136
ccgtcgctca ggatggtaac                                                    20

<210>  137
<211>  20
<212>  DNA
<213>  Artificial Sequence

<220>

<223>  primer

<400>  137
gcgcttccga agtagtggaa                                                    20


<210>  138
<211>  25
<212>  DNA
<213>  Artificial Sequence

<220>
<223>  probe

<400>  138
accgggccag cggcatctac tgctt                                              25